# EUROPEAN PATENT APPLICATION

(11) **EP 3 816 905 A1**
(43) Date of publication of application: **05.05.2021**
(21) Application number: 19827094.4
(22) Date of filing: 21.06.2019
(51) Int. Cl.: G06Q 10/10, A61B 5/16, G09B 19/00

(54) **VISUAL APPROACH-BASED APTITUDE TESTING SYSTEM**

(30) Priority: 27.06.2018 JP 2018121570
(71) Applicant: Jinsoken Co., Ltd., Chuo-ku, Tokyo 103-0006 (JP)
(72) Inventor: NISHIOKA Koichi, Tokyo 103-0006 (JP)
(74) Representative: TBK
(86) International application number: PCT/JP2019/024718
(87) International publication number: WO 2020/004263

(57) **Abstract**

[Problem] To grasp human mental structure in a multifaceted manner through a visual information, and to accurately and objectively grasp an examinee's propensity such as a vocational aptitude.

[Solution] The potential consciousness of an examinee is expressed by the application state of the multiple icons in a check sheet comprising a large number of areas and figures having no particular meaning by itself in a frameless test area. It is possible to visually and specifically express an examinee's internal and potential consciousness through the images obtained from the examinee's application state of icons on a check sheet. Amygdala that controls emotion will respond to visual information responds 100ms earlier than neocortex. Therefore, it is possible to understand an examinee's aptitude by grasping his/her subconsciousness under unawareness prior to conscious controls.
Since the icons are recognized as figures, they affect subconscious of the frontal lobe without stimulating the emotional system.

## Description

### Technical field

The present invention relates to a system for multifacetedly grasping and judging human psychiatric disciplines through visual information, and more particularly, to a visual approach-based aptitude testing system for judging how well an examinee is qualified for a particular occupation.

### Background Art

Human behavior is a cross-over of various impacts derived from psychological situations and has relationship with basic potential and performance potential. Various external stimuli may act as a burden when engaging in business, and psychosomatic stress may occur in such cases. Since stress is invisible in most cases, it is often difficult to visually and accurately grasp the business-related stress.

In addition, concerning how to evaluate human performance in business, concentration (ability to work) and wage differentials derivable therefrom (rewarding money) has been considered from the viewpoint of market value.

However, since the conventional performance theory is a rigid structural system in which the analytical result is difficult to be reused, it is difficult to make an appropriate determination from the results of aptitude test based on the theory using said system.

By the way, there is a diversity in people's social behavior. The frontal lobes have been said to suppress the selfish old brain, as to why diverse people act with cooperation.

Recently, however, the theory that emotion system avoids inequity has become dominant. More specifically, the predictive accuracy of purchasing behavior, IQ estimates from resting fMRI, and MRI-based predictions indicate that people have a mechanism to avoid unfairness, and amygdala, that is an older brain in the deepest part, reacts intuitively and automatically to avoid unfairness and behave in a prosocial manner.

Prosocial behavior refers to the one that maximizes the harmony between oneself and others and minimizes differences therebetween, which will differ from individualistic behavior that maximizes oneself and also from competitive behavior that maximizes differences between oneself and others.

Analysis of fMRI data shows that the absolute value of reward difference and the activity of amygdala are correlated with each other in prosocial behavior, and amygdala of prosocial becomes active when there is a large absolute value of the reward difference.

The results of the final proposed game experiment and the trust game experiment support the above theory, and there is a possibility that social disparity and depression tendency are related with each other.

As a result of fMRI analysis, it has been proved that prefrontal area (frontal association area) or neocortex, which is evolutionarily new and constitutes the center of high order cognitive function, expresses guilt, and evolutionarily primitive amygdala and nucleus accumbens express inequality.

In addition, 1/3 of the cerebral cortex is related to the visual sense.

Traditionally, it has been said that competition creates progress (Eiichi Shibuya); only comparing yourself with others is a social slave (Rousseau); and comparing with others is the shortest path to become unfortunate (Buddha).

However, recent dominant theories have been reported that social disparities are correlated with depressive tendencies.

Summarizing the above, recent advances in brain function imaging technologies have revealed that some kind of human social decision-making in brain is based primarily on subcortical area and cerebral cortex.

In addition, frontal lobe (neocortex) relates to conscious information processing, amygdala relates to emotional information processing such as fear and pleasure, and septum and hippocampus relates to memory and emotion control. Considering the structures of the brains, people have two minds, one is conscious, determinable mind, mainly existing in the frontal lobe, and the other is unconscious emotion, mainly existing in amygdala.

For some subconscious and apparent awareness, perceptual and behavioral determinations, including human subjective consciousness, are known to be made in frontal lobe (neocortex). On the other hand, emotion (response of the amygdala) is not directly conscious.

The degree of development of the frontal lobe (neocortex) differs between adults and children. In other words, the response by frontal lobe (neocortex) is considered to be low for children. Actually, a human perceives the result of expression of emotion to the human body, and recognizes one's emotion by comparing it with previous experiences, and expresses the same as emotions. In addition, it is said that some behaviors are not conscious as in custom, but are reflexive.

The main Literature referred to by the present invention are as follows.
<1> Concerning the evolution model from the degree of psychological demands and determination making: Non Patent Literature 1
<2> Concerning stress observation: Non-Patent Literature 2, 3, and 4
<3> Concerning edge effect: Non Patent Literature 5 and 6
<4> Concerning moiré phenomenon: Non Patent Literature 7, 8, and 9
<5> Concerning the performance of human beings: Non Patent Literature 10 and 11
<6>Stress Theory: Non Patent Literature 12, 13, and 14
<7> Psychological and physiological approaches to emotions: Non Patent Literature 15, 16, and 17
<8> General adaptation syndrome (GAS) Theory: Non Patent Literature 18
<9> Information from brain science, cognitive science and neuroscience: Non Patent Literature 19

### Citation List

### Non Patent Literature

[Non Patent Literature 1] Psychological demand/decision latitude model: Karasek and Theorell (1990)
[Non Patent Literature 2] Stress Viewer (model):by Johanna Paula Monique Fikkert, Haike Jacobs - Language Arts & Disciplines - 2003 - 463 pages
[Non Patent Literature 3] 1995 Metrical Stress Theory: Principles and case studies.... Hogg, Richard 1992
[Non Patent Literature 4] A Grammar of Old English, volume I: Phonology, Oxford: Basil Blackwall....
[Non Patent Literature 5] Edge effect and job stress (theory): Cross-Functional Project Groups in Research and New Product Development; Diversity, Communications, Job Stress, and Outcomes
[Non Patent Literature 6] Robert T. Keller The Academy of Management Journal, Vol. 44, No. 3 (Jun., 2001), pp. 547-555
[Non Patent Literature 7] Stress moire (theory): A Water-Bonded, Floating Element Shear-Stress Sensor Using a Geometric Moire Optical Transduction Technique
[Non Patent Literature 8] Stephen Horowitz, Tai-An Chen, Venkataraman Chandrasekaran, Ken Tedjojuwono, Louis Cattafesta, Toshikazu Nishida, and Mark Sheplak
[Non Patent Literature 9] Interdisciplinary Microsystems Group, PO Box 116250, University of Florida, Gainesville, Florida 32611-6250
[Non Patent Literature 10] Human Performance(model): Enhancing Human Performance: Background Papers, Stress Management
[Non Patent Literature 11] Authors: Committee on Techniques for the Enhancement of Human Performance, National Research Council
[Non Patent Literature 12] Stress theory and phonology in Old and Middle English Keiko Kaminashi (1994)
[Non Patent Literature 13] Managing Stress: Principles and strategies for Health and Wellbeing by Brian Luke Seaward - Self-Help - 2005 - 590 pages
[Non Patent Literature 14] Lazarus, RS, and Folkman, S. Stress, Appraisal, and Coping. Springer, New York, 1984. Matheny, K.., et al. Stress Coping: A Qualitative and Quantitative ...
[Non Patent Literature 15] Psychological and Biological Approaches to Emotion: by Nancy L. Stein, Bennett Leventhal, Tom Trabasso - Psychology - 1990
[Non Patent Literature 16] Stress, appraisal, and coping. New York: Springer, Lazarus, RS, Kanner, AD, & Folkman, S. (1980).
[Non Patent Literature 17] Emotions: A cognitive-phenomenological analysis....
[Non Patent Literature 18] Stress without Distress (1974), Stress of Life (1956) by Hans Hugo Bruno Selye CC
[Non Patent Literature 19] October 30, 2017, Consortium for Applied Neuroscience, Masahiko Haruno, NICT Brain-Information and Communications Centre.

### Summary of Invention

### Technical Problem

In view of the above-mentioned background, an object of the present invention is to grasp human mental structure in a multifaceted manner through a visual information, and to accurately and objectively grasp an examinee's propensity such as a vocational aptitude.

### Solution to Problem

To achieve the above object, a visual approach-based aptitude testing system according to the present invention is an aptitude testing system comprising a headquarter computer connected to an examinee's terminal through a communication network;
said headquarter computer includes:
means for providing, to said examinee's terminal, a check sheet in which a plurality of areas are formed by boundary elements depicted in a frameless test area and which comprises graphic figures having no particular meaning by itself, and a plurality of icons applicable to said check sheet;
storage means for storing table 1 which predetermines the definition of said boundary elements in said check sheet, table 2 which predetermines the kind, number and definition of said icons, and table 3 which predefines the meaning of position of said icon in said check sheet;
writing means for sequentially writing, to said storage means, test data including said icons applied by said examinee and an examinee's ID transmitted from said examinee's terminal;
count-up execution means for sequentially reading out said test data and said examinee's ID stored in said storage means to count the total number of said icons;
means for executing discrimination whether or not the arrangement of said icons in said read-out test data falls under said definition in each tables, wherein the results of said discrimination are stored in said headquarter computer and used for evaluation of the examinee's aptitude based on the total number of said icons in a test object and the overall shape of the test object.

A visual approach-based aptitude testing system according to claim 2 of the present invention is characterized in that, in the visual approach-based aptitude testing system according to claim 1, wherein said boundary elements comprise lines and/or corners.

A visual approach-based aptitude testing system according to claim 3 of the present invention is characterized in that, in the visual approach-based aptitude testing system according to claim 2, wherein said lines comprise straight lines, curved lines and broken lines or any combinations thereof.

A visual approach-based aptitude testing system according to claim 4 of the present invention is characterized in that, in the visual approach-based aptitude testing system according to claim 2 or 3, wherein said corners comprise corners of virtual frame, and/or points of intersection between lines.

A visual approach-based aptitude testing system according to claim 5 of the present invention is characterized in that, in the visual approach-based aptitude testing system according to any one of claims 1 to 4, wherein said storage means further stores table 4 which predefines the meaning of positional relationship (hereinlater referred to by "icon relationship") between a plurality of said icons or between said icon and said line, and said headquarter computer further comprises means for executing discrimination whether or not said icon relationship exists in the arrangement of said icons in said read-out test data, in reference to said definition in each of said tables.

A visual approach-based aptitude testing system according to claim 6 of the present invention is characterized in that, in the visual approach-based aptitude testing system according to any one of claims 1 to 5, wherein said storage means further stores table 5 which predefines the meanings of said applied icons forming a specific pattern, and said headquarter computer further comprises means for executing discrimination whether or not said predefined specific pattern exists.

A visual approach-based aptitude testing system according to claim 7 of the present invention is characterized in that, in the visual approach-based aptitude testing system according to any one of claims 1 to 6, wherein said storage means further stores table 6 which predefines the meaning of appearance of a specific one of said icons, and said headquarter computer further comprises means for executing discrimination whether or not said specific icon appears.

A visual approach-based aptitude testing system according to claim 8 of the present invention is characterized in that, in the visual approach-based aptitude testing system according to claim 6, wherein said specific pattern comprises crowded arrangement, dispersive arrangement, balanced arrangement, deviative arrangement, bottom arrangement, upper arrangement, top-and-bottom arrangement, left-and-right arrangement, protruding arrangement and /or doll arrangement.

A visual approach-based aptitude testing system according to claim 9 of the present invention is characterized in that, in the visual approach-based aptitude testing system according to claim 5, wherein said icon relationship comprises overlapping, contact, line-contact and/or close-vicinity.

### Advantageous Effects of Invention

According to the visual approach-based aptitude testing system of the present invention, it is possible to visually and specifically express the examinee's internal and potential consciousness through the images obtained from the application state of icons on a check sheet, more particularly the number and position of the icons applied by an examinee. Thus, the examinee's mental structure can be grasped in a multifaceted manner through vision.

As mentioned above, one-third of the cerebral cortex is involved in vision. To visual stimuli i.e. visual information, an amygdala that is the emotional brain controlling emotion will respond faster than a neocortex which is said to conduct cognitive processing. Amygdala response to the stimulus takes place in 100ms after receiving the stimulus, 100ms before neocortex response which takes place in 200ms after receiving the stimulus.

The present invention takes advantage of this amygdala's rapidity of response times to express subconscious under unawareness. This is because the examinee's potential consciousness can be grasped prior to correction by neocortex (control under awareness), thereby demonstrating the examinee's aptitude.

In other words, the icons are perceived visually (i.e., with one's eyes) and at the same time stimulate amygdala belonging to older brains. The stimulation to amygdala precedes the stimulation to the frontal lobe.

The information that affects subconscious includes the response of the amygdala (emotion response), in addition to the information from the sensory organs sensed by neocortex (frontal lobe). Since the response by the neocortex differs from the response by the amygdala by 100ms, there are two types of subconscious, one being affected by the amygdala's response and the other being less affected by the amygdala's response.

Also, icons, even human icons do not have facial expressions, but are recognized as graphics. Thus, the icons affect subconscious of the frontal lobe without stimulating emotional system.

The icon examination invokes this frontal lobe subconscious, and the icon application operation is expressed as a response thereto.

The amygdala, which reacts in 100ms, will less affect the frontal lobe in its immediate response, but the cumulative emotion may affect the frontal lobe, and this difference appears in a manner how the icons are applied. The present invention utilizes the time lag of this response.

This is the theoretical basis of the present invention based on brain science, cognitive science, neuroscience, and the like, and the present invention grasps the examinee's career aptitude and propensity based on this basis. As described above, according to the present invention, since the examinee's internal and potential consciousness can be expressed through the visual sense, it is possible to grasp the examinee's mental structure in a multifaceted manner. Therefore, the test results can be directly used to evaluate the examinee's personality and aptitude.

According to the visual approach-based aptitude testing system according to claims 2 to 4, the bounding elements may be determined variously or in a simplified manner, so a variety of investigations and analyses can be carried out, ranging from complicated investigations to simple investigations with less burden on the examinee.

According to the visual approach-based aptitude testing system according to claims 5 to 7, since it is also possible to determine the icon relationship, the specific pattern, and the specific icon, it is possible to more accurately grasp the examinee's aptitude and propensity.

According to the visual approach-based aptitude testing system of claim 8 and/or claim 9, since the specific pattern and the icon relationship can be determined more accurately, it is more effective for grasping the examinee's aptitude and propensity.

### Brief Description of Drawings

[Fig. 1A] It is a screen showing embodiments of check sheets and icons used for the visual approach-based aptitude testing system according to the Invention.
[Fig. 1B] It is a figure for explaining Fig. 1A;
[Fig. 2A] It is a figure showing a template of lines, corners, areas and contact points described in the check sheet of Fig. 1.
[Fig. 2B] It is a conversion table of the template.
[Fig. 2C] It is a continuation of Fig. 2B;
[Fig. 3A] It is a code table showing the meaning of each icon and the display when each icon is placed on each line, each contact point, each corner, and in each area.
[Fig. 3B] It is a continuation of drawing 3A.
[Fig. 3C] It is a continuation of Fig. 3B.
[Fig. 3D] It is a continuation of Fig. 3C.
[Fig. 3E] It is a continuation of Fig. 3D.
[Fig. 3F] It is a continuation of Fig. 3E.
[Fig. 3G] It is a continuation of Fig. 3F.
[Fig. 3H] It is a continuation of Fig. 3G.
[Fig. 3I] It is a continuation of Fig. 3H.
[Fig. 3J] It is a continuation of Fig. 3I.
[Fig. 3K] It is a continuation of Fig. 3J.
[Fig. 3L] It is a continuation of Fig. 3K.
[Fig. 4A] It is a figure showing the meaning of "overlapping" of the icons.
[Fig. 4B] It is a continuation of Fig. 4A.
[Fig. 4C] It is a continuation of Fig. 4B.
[Fig. 4D] It is a continuation of Fig. 4C.
[Fig. 5A] It is a code table (single) of logic comments of the visual approach-based aptitude testing system according to the present invention.
[Fig. 5B] It is a continuation of Fig. 5A.
[Fig. 5C] It is a continuation of Fig. 5B.
[Fig. 6] It is a code table (overlapping) of logic comments of the visual approach-based aptitude testing system according to the present invention.
[Fig. 7A] It is a master sheet for test analysis by the visual approach-based aptitude testing system according to the present invention.
[Fig. 7B] It is a continuation of Fig. 7A.
[Fig. 7C] It is a continuation of Fig. 7B.
[Fig. 8] It is a master sheet for a headquarter computer of the visual approach-based aptitude testing system according to the present invention.
[Fig. 9A] It is a figure showing a calculation logic of the crowded arrangement.
[Fig. 9B] It is a figure showing a calculation logic of the dispersive arrangement.
[Fig. 9C] It is a figure showing a calculation logic of the deviative arrangement.
[Fig. 9D] It is a figure showing a calculation logic of the bottom arrangement.
[Fig. 9E] It is a figure showing a calculation logic of the upper arrangement.
[Fig. 9F] It is a figure showing a calculation logic of the protruding arrangement.
[Fig. 9G] It is a figure showing a calculation logic of the doll arrangement".
[Fig. 10] It is a figure showing criteria separately provided for determining the details of the doll arrangement, including (A) showing standard specification and (B) showing examples of samples.
[Fig. 11] It is a figure showing the process specifications of the results by the visual approach-based aptitude testing system according to the present invention.
[Fig. 12] It is a process flow chart illustrating an example of the visual approach-based aptitude testing system according to the present invention.
[Fig. 13] (A) shows an example of the visual approach-based aptitude testing system according to the present invention, which is the figure showing scanner input images of icons applied to the check sheet by an examinee, and (B) is a figure showing locations of input data by (A) in the computers.
[Fig. 14] (A) shows another example of the visual approach-based aptitude testing system according to the present invention, which is the figure showing scanner input images of icons applied to a check sheet by an examinee, and (B) is a figure showing locations of input data by (A) in the computers.
[Fig. 15] (A) shows another example of the visual approach-based aptitude testing system according to the present invention, which is the figure showing scanner input images of icons applied to the check sheet by an examinee, and (B) is a figure showing locations of input data by (A) in the computers.
[Fig.16] (A) shows another example of the visual approach-based aptitude testing system according to the present invention, which is the figure showing scanner input images of icons applied to the check sheet by an examinee, and (B) is a figure showing locations of input data by (A) in the computers.
[Fig. 17] (A) shows another example of the visual approach-based aptitude testing system according to the present invention, which is the figure showing scanner input images of icons applied to the check sheet by an examinee, and (B) is a figure showing locations of input data by (A) in the computers.
[Fig. 18A] It is a figure showing an application embodiment of the crowded arrangement.
[Fig. 18B] It is a figure showing an application embodiment of the dispersive arrangement.
[Fig. 18C] It is a figure showing an application embodiment of the balanced arrangement.
[Fig. 18D] It is a figure showing an application embodiment of the deviative arrangement.
[Fig. 18E] It is a figure showing an application embodiment of the bottom arrangement.
[Fig. 18F] It is a figure showing an application embodiment of the upper arrangement.
[Fig. 18G] It is a figure showing an application embodiment of the top-and-bottom arrangement.
[Fig. 18H] It is a figure showing an application embodiment of the left-and-right arrangement.
[Figure 18I] It is a figure showing an application embodiment of the protruding arrangement.
[Fig. 18J] It is a figure showing an application embodiment of the doll arrangement.
[Fig. 18K] It is a figure showing an application embodiment of the overlapping.
[Fig. 18L] It is a figure showing an application embodiment of the contact.
[Fig. 18M] It is a figure showing an application embodiment of the close-vicinity.
[Fig. 19A] It is a screen showing another embodiment of the check sheet used for the visual approach-based aptitude testing system according to the present invention.
[Fig. 19B] It is a figure illustrating a template of lines, corners, areas and contact points described in the check sheet of Fig. 19A.
[Fig. 20] It is a screen showing another embodiment of the check sheet used for the visual approach-based aptitude testing system according to the present invention.
[Fig. 21] It is a screen showing another embodiment of the check sheet used for the visual approach-based aptitude testing system according to the present invention.

Next, visual approach-based aptitude testing system according to the present invention will be described in more detail with reference to the drawings showing the embodiment of the present invention. For the sake of convenience, the same reference numerals are assigned to portions which perform the same functions, and descriptions thereof are omitted.

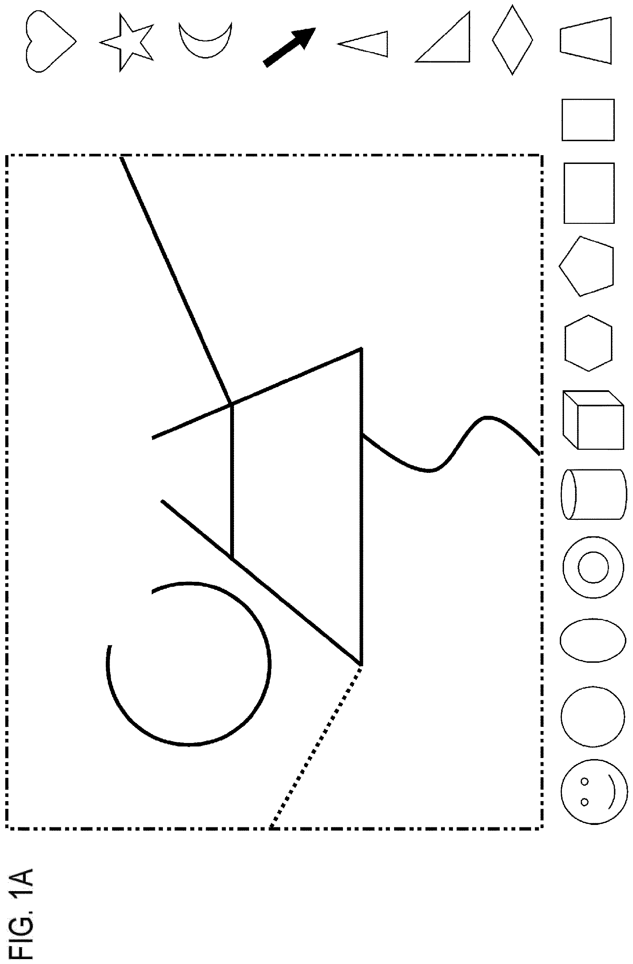

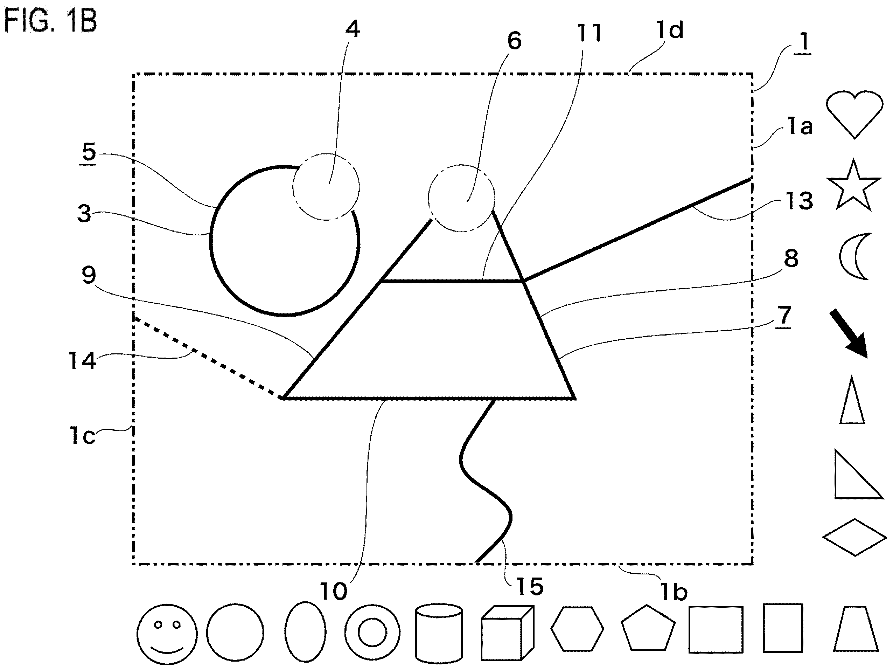

Fig. 1 shows check sheets and icons used in a visual approach-based aptitude testing system in accordance with the present invention. In this embodiment, the areas formed by combining the boundary elements (described later) are depicted in a frameless test area, and it is a so-called open sheet. In this case, the areas shown in a display can be grasped as a window when the check sheet is considered to be a part of a spherical surface. For the sake of convenience, this window is shown by a two-dot chain line in Fig. 1 and the like. The check sheet includes areas comprising a number of circular figures and polygonal figures wherein straight lines, curved lines and broken lines are suitably combined. These form a closed space, an open space, an open circle space, a partly-open trapezoidal space, a slope and a specifically-defined space. An examinee operates his/her portable terminal such as a smartphone to apply icons on an image of the check sheet transmitted from a computer (not shown). There are 18 types of icons, which will be described later in more detail. The headquarter computer stores the same figures and areas as the check sheet and icons of Fig. 1.

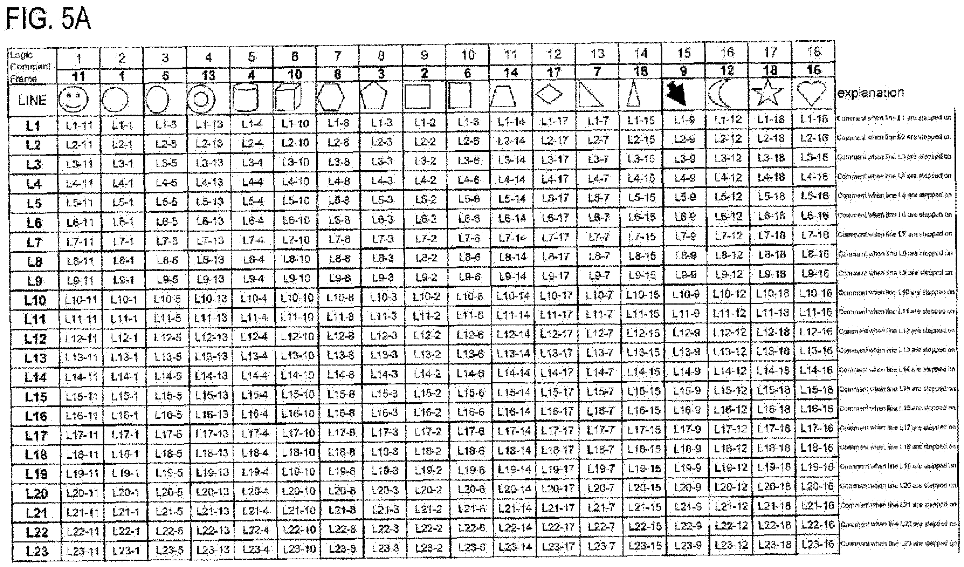

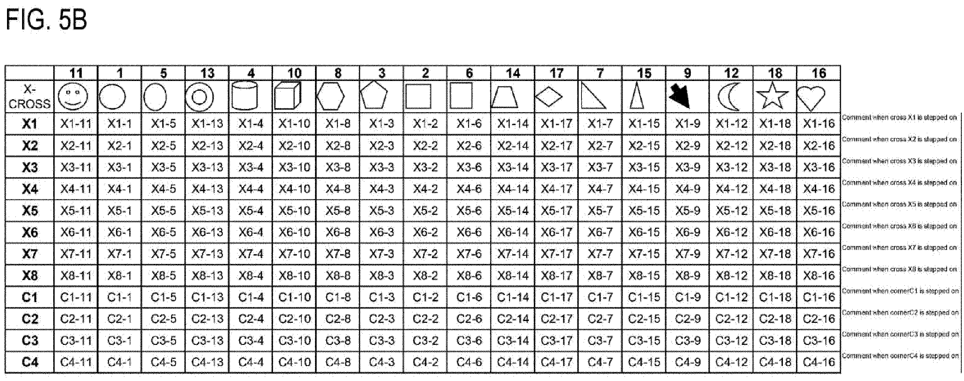

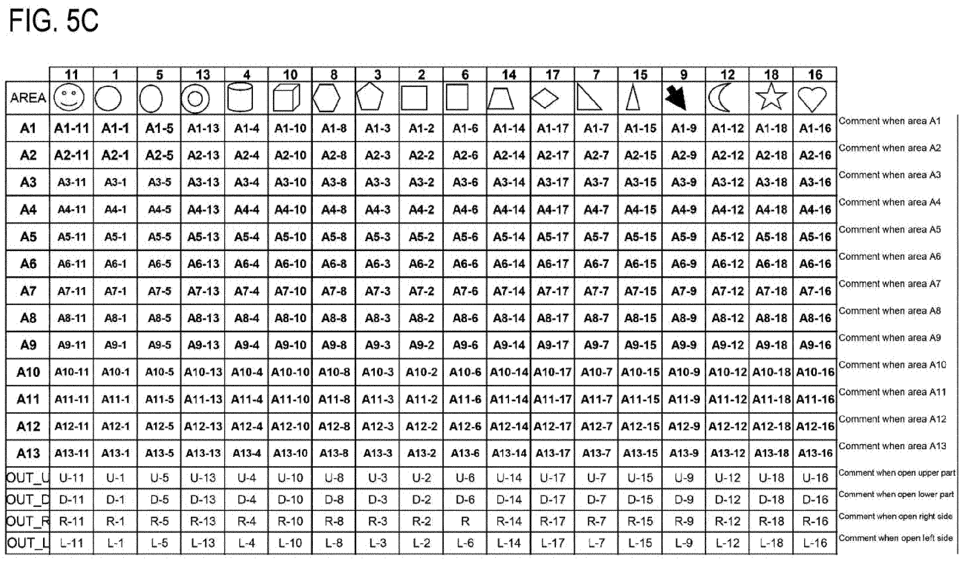

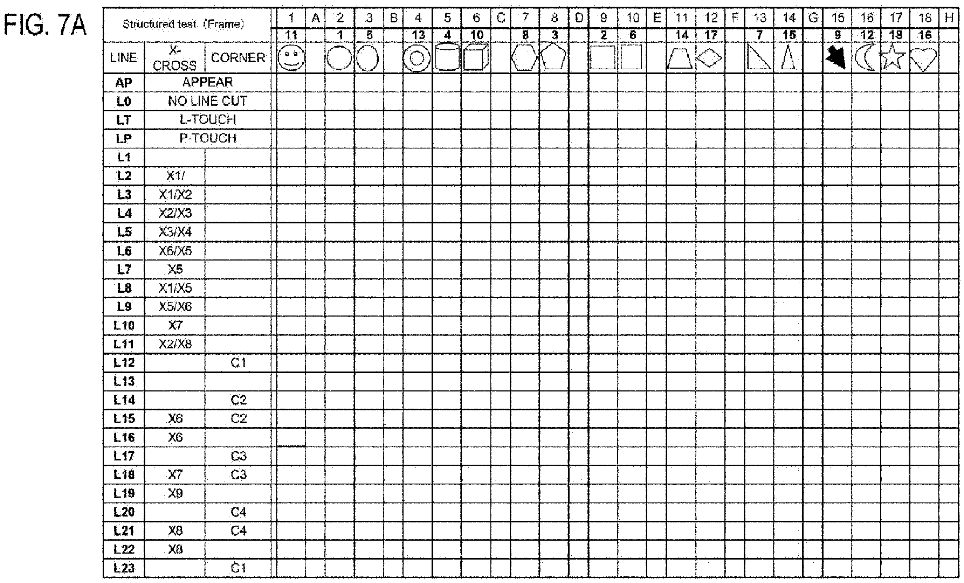

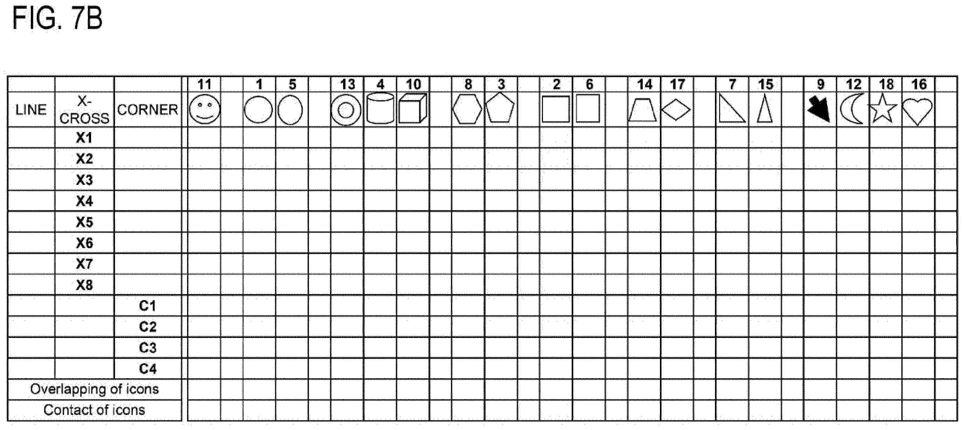

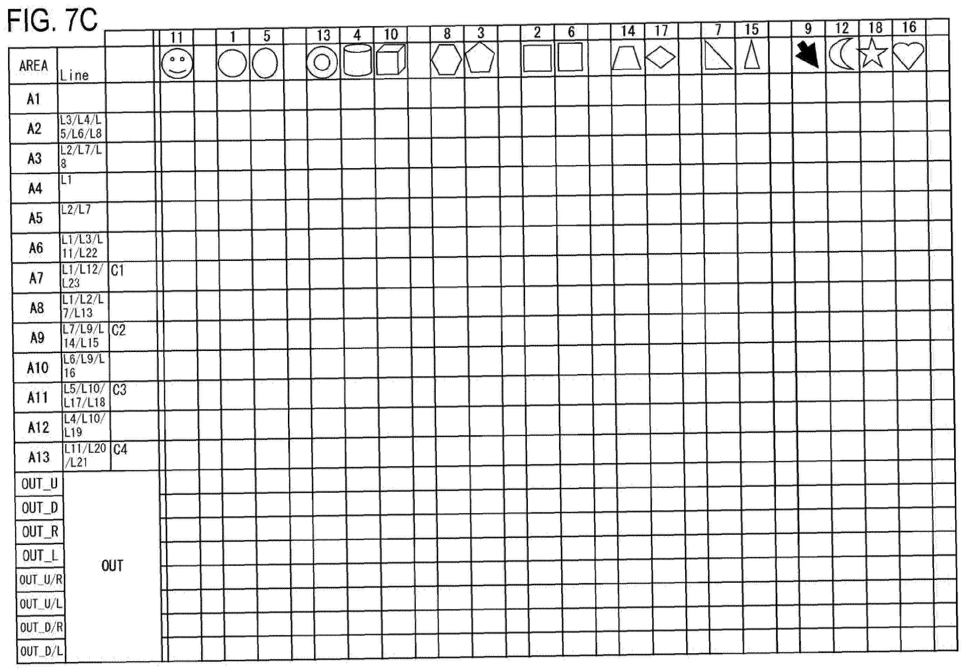

Next, the boundary elements shown in Fig. 1 is outlined. More detailed description is given below in the following paragraphs. Different types of the boundary elements are depicted in window 1 shown by a two-dot chain line for the sake of convenience. The boundary elements comprise a circular figure 5 having a cut-off 4 at the upper-right part of the circumferential curved line 3, a triangular figure 7 having a cut-off 6 at the apex thereof and an intermediate straight line 11 extending in parallel with a straight bottom line 10 between opposite oblique-side lines 8, 9, a straight line 13 (inclined straight line) extending obliquely up and to right from the right end of the intermediate line 11 to the window virtual line 1a, a broken line 14 (inclined broken line) extending obliquely up and to left from the left end (opposite to the inclined straight line 13) of the bottom 10 to the window virtual line 1c, and a S-shape curved line 15 extending down from the rightward middle portion of the bottom 10 to the window virtual line 1b. With these boundary elements, the areas to be described in detail below are divided into nine sectional areas, among which three are located almost in a horizontal direction and other three almost in a vertical direction. Both cut-offs 4 and 6 open upward. Figure comprising any combination of said boundary elements have no complete meaning in itself. Many areas sectioned in the check sheet are formed by said boundary elements.

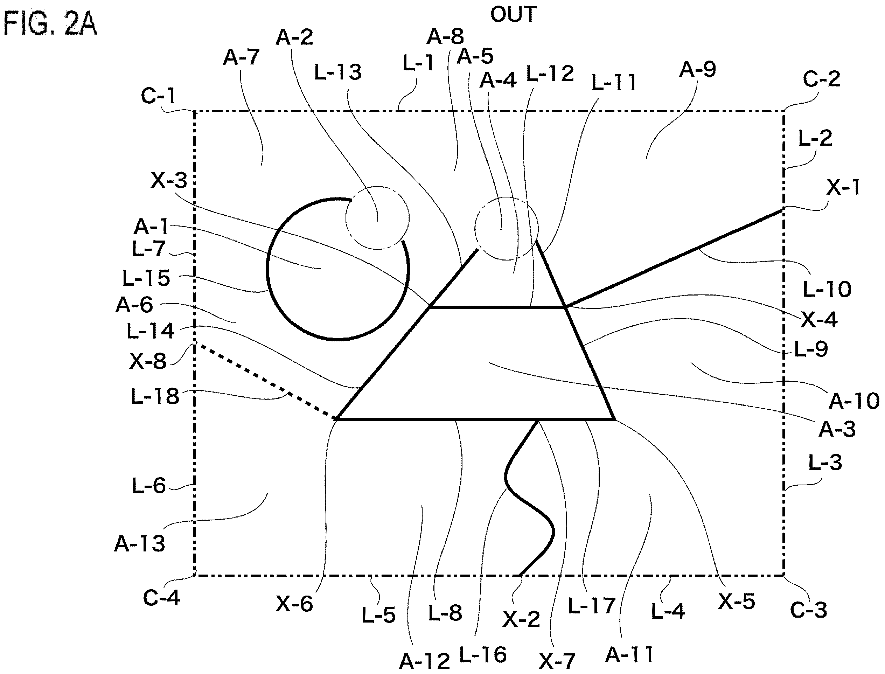

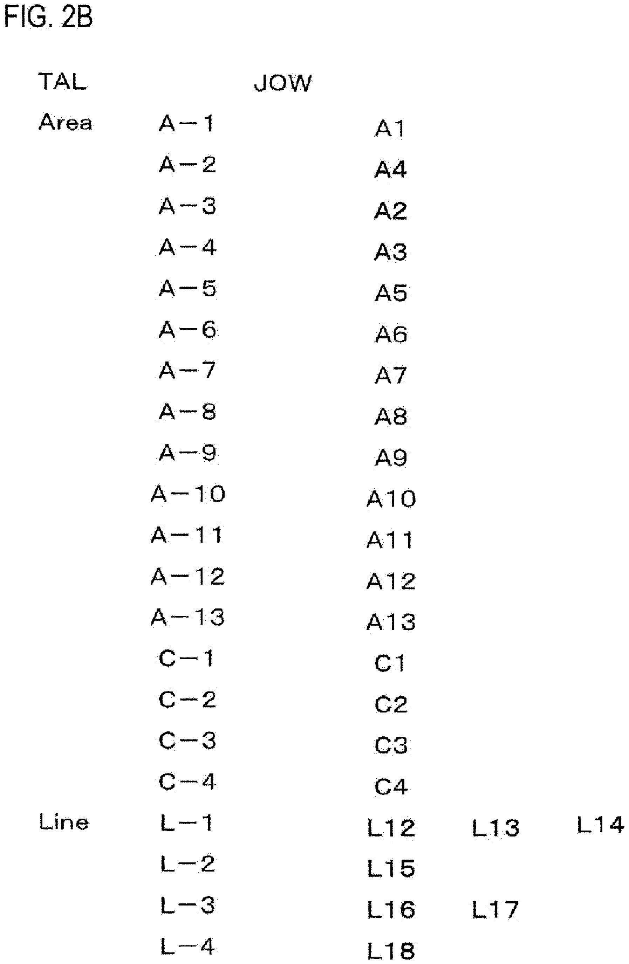

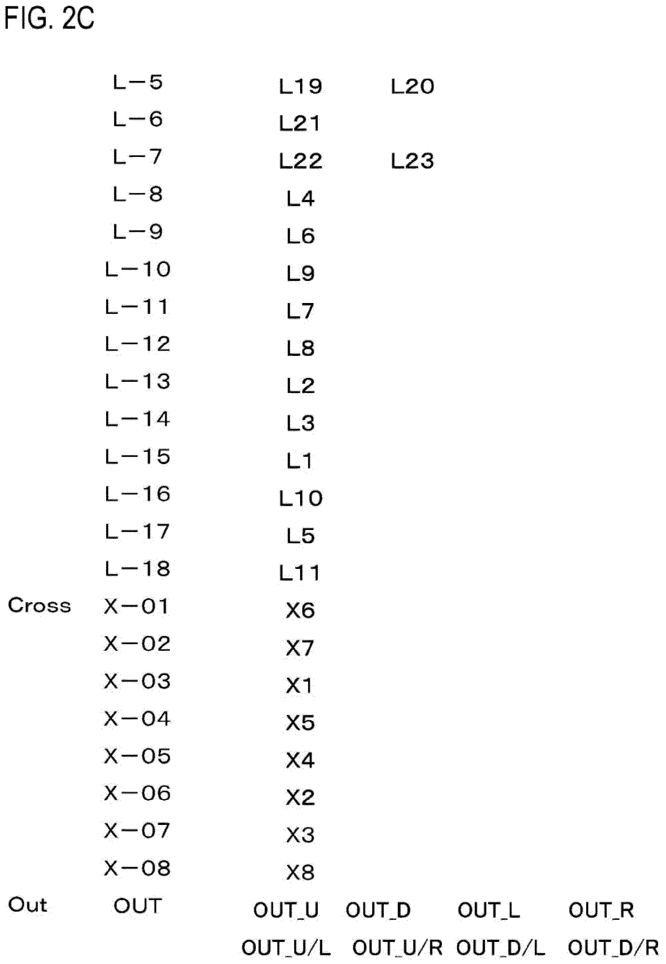

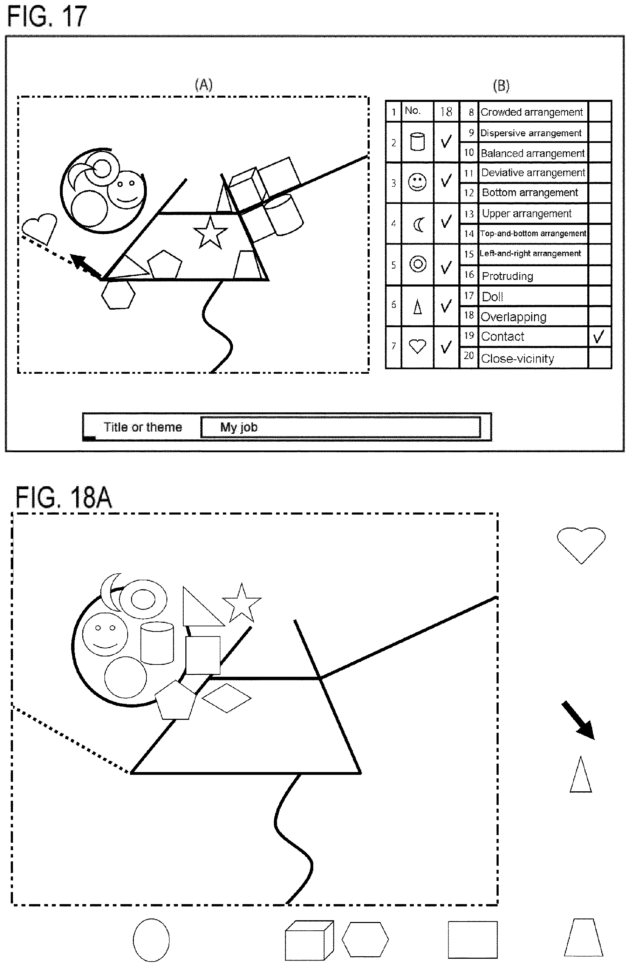

Figure 2A shows templates of lines, corners, areas and contact points described in the check sheet, and Figure 2B and 2C show their conversion tables. TABLE 1A shows lines L1-L18. TABLE 1B shows corners C1-C4. TABLE 1C shows areas A1 to A13. TABLE 1D shows contact points X1-X8. TABLE 2 shows the shape, name and color of each icon. TABLE 3A and TABLE 3B show relationships between icons and their meanings in each of the A-H systems. TABLE 4 shows definition of the meaning of the total number of the icons selected. TABLE 5 shows definition of specific pattern (described later) to be grasped in specific situation. TABLE 6 shows definition of interpretations of these specific patterns. TABLE 7 is a template indicating meaning of icons (described later). Information of Figs. 1 to 11 (described later), Figs. 18A to 18M and TABLES 1A to 8 are stored in advance in the headquarter computer.

Here, the contents of TABLES 1A to 8 will be described. TABLE 8 shows relationship among table 1 to table 6, the individual TABLES and the drawings. The specified comments in TABLE 3A, TABLE 3B, TABLE 4, TABLE 5 and TABLE 6 are based on the findings statistically obtained from the test results.

**[TABLE1A]**

| Line Number | Position of the Line |
|---|---|
| L-1 | A line at the upper side of rectangle chart |
| L-2 | Upper part of the line on the right-hand side of rectangle chart |
| L-3 | Bottom of the line on the right-hand side of rectangle chart |
| L-4 | Right of the line at the lower side of rectangle chart |
| L-5 | Left of the line at the lower side of rectangle chart |
| L-6 | Bottom of the left-hand side of rectangle chart |
| L-7 | Upper part of the line on the left-hand side of rectangle chart |
| L-8 | Left of the line at the bottom of trapezoid diagram in the center of the chart |
| L-9 | A line on the right-hand side of trapezoid diagram in the center of the chart |
| L-10 | Line inclined rightward and upward divided the upper part and bottom of rectangle chart |
| L-11 | A line on the right-hand side of a triangular diagram drawn at the center of a chart |
| L-12 | A line at the bottom of a triangular diagram drawn at the center of a chart |
| L-13 | A line on the left-hand side of a triangular diagram drawn at the center of a chart |
| L-14 | A line on the left-hand side of trapezoid diagram in the center of the chart |
| L-15 | A line that is an arc of a circle lacking a part of the circumference drawn on the left side of the chart |
| L-16 | Line of S curved line that divides the area A-11 and the area A-12 drawn at the bottom of the chart |
| L-17 | Right side of the line that forms the bottom of trapezoid diagram drawn in the center of the chart |
| L-18 | A line of broken line that divides the upper part and bottom of the chart on the left side join the intersection X-6 and the intersection X-8. |

**[TABLE 1B]**

| Corner Number | Position of the Corner |
|---|---|
| C1 | Upper left part |
| C2 | Upper right part |
| C3 | Lower right part |
| C4 | Lower left part |

**[TABLE 1C]**

| Area Number | Shape of the Area |
|---|---|
| A-1 | An area consisting of a circle with a missing upper right corner located in the upper left corner of the chart |
| A-2 | The part of the missing arc in the area A-1 (upper right part) |
| A-3 | An area consisting of trapezoid located in the center of the chart |
| A-4 | Area consisting of a top-missing triangle connected to the upper part of the A-3 area |
| A-5 | The upper part missing A-4 area |
| A-6 | An area that the upper left part is divided integrally with the area A-7 and the upper right part is divided integrally with the area A-8, a circular arc is formed in a part of the upper part, and consisting heteromorphic figure of a broken line which lower line is inclined |
| A-7 | The area that divided as an integral area of each area of the areas A-8 and A-9, left side of the area formed in the upper part of the chart, and circular arc is located in the lower right side and lower left part adjoins A-6 Area. |
| A-8 | The area that divided as an integral area of each of the areas A-7 and A-9, the center of the area formed in the upper part of the chart, and the area A-5 is located in the lower part. |
| A-9 | The area that divided as an integral area with the areas A-7 and A-8, the right side of the area formed in the upper part of the chart, a line L-10 consisting of a right-up inclined line is formed in the lower part, and a line L-11 consisting of a left-up inclined line is formed in the left side. |
| A-10 | It is the upper part area divided as an integral area with A-11, a line L-10 consisting of an inclined line rising to the right is formed at the upper end, and a line L-9 consisting of an inclined line rising to the left is formed at the left end |
| A-11 | It is a lower area divided as an integral area with A-10, and a line L-16 made of S curved line is formed at the left end, and a line L-17 is formed at the upper left end. |
| A-12 | It is a right side area divided as an integral area with A-13, and a line L-16 made of S curved line is formed at the right end portion. |
| A-13 | It is a left side area divided as an integral area with A-12, and a line L-18 composed of a broken line is formed at the upper end. |

**[TABLE 1D]**

| Contact Point Number | Position of the contact point |
|---|---|
| X-1 | Intersection of line L-2 and line L-10 |
| X-2 | Intersection of line L-4 and line L-16 |
| X-3 | Intersection of line L-13 and line L-12 |
| X-4 | Intersection of line L-11 and line L-12 |
| X-5 | Intersection of line L-9 and line L-17 |
| X-6 | Intersection of line L-14 and line L-8 |
| X-7 | Intersection of line L-8 and line L-16 |
| X-8 | Intersection of line L-7 and line L-18 |

**[TABLE 2]**

| Icon Number | Shape/Name of the Icon | Color |
|---|---|---|
| 1 | Circle | Pink |
| 2 | Square | Light gray |
| 3 | Regular pentagon | Purple |
| 4 | Column | Green |
| 5 | Ellipse | Light yellow |
| 6 | Rectangle | Light blue |
| 7 | Right triangle | Yellow |
| 8 | Regular hexagon | Orange |
| 9 | Arrow | Dark gray |
| 10 | Cube | Light pink |
| 11 | Face (circle and two eye-like small circles in the circle and mouth-like concave arcuate surface) | White (gray for small circles only) |
| 12 | Moon (arcuate surface) | Gray |
| 13 | Doughnut (concentric circle surface) | Light yellow green |
| 14 | Trapezoid | Light blue |
| 15 | Isosceles triangle | Dark pink |
| 16 | Heart shape | Blue |
| 17 | Rhomboid | Ivory |
| 18 | Star | Yellow |

**[TABLE 3A]**

| | Number | | Icon | Overview of Icons' Meanings | Specified Comment |
|---|---|---|---|---|---|
| A | | Face system | | | |
| 1 | **11** | | 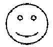 | It represents relationships including people (self and others). The inclination of the icons represents the bias of thought, and the reversal represents self-denial or neglect of others. If it exists in enclosed space (A3), it represents isolation or uniqueness, and contact with other icons requires a separate meaning search. | Want to be related to people. Have someone who I want to be targeted. Cannot ignore people. Have someone important. Have someone dislike. |

| B | | ○ System | | | |
|---|---|---|---|---|---|
| 2 | **1** | | 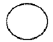 | It It shows soft objects. It expresses peace and satisfaction, and it expresses ease of cooperativeness. Contact with a line of the check sheet represents a vague request and sense of security. If it exists in enclosed space, it somehow expresses one's feelings of loneliness. | Kindness. Warmness. Calm. I'm not be nervous. Gentle. |
| 3 | **5** | | 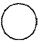 | It It shows soft objects. It represents peace and satisfaction, and expresses flexibility in cooperativeness. Contact with a line of the check sheet represents a vague request and sense of security If it exists in enclosed space, it somehow represents a feeling of loneliness that is not just oneself. The inclination of the icons represents the instability of thought, and dependence may be strong. If it is selected, it has a somewhat unique aspect. | Soft. Good person. People (humans). Eggs, lukewarm. Can feel easy. |

| C | Three-dimensional system | | | | |
|---|---|---|---|---|---|
| 4 | **13** | | 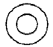 | It shows soft objects. Though it represents peace and satisfaction, cooperativeness with others is thin. Contact with a line of the check sheet represents a vague despondency and sense of security. If it exists in enclosed space, there may be some strong claims of loneliness of one's own. The combination with the icons (9: arrow) requires a strong sense of fulfillment. | Strange feeling. With holes. Want to escape. Want to hide. Move. Car. Run. |
| 5 | **4** | | 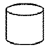 | It makes people aware of its strong presence, but it does not have a very deep meaning. This is often the case because of the interesting nature of the icons. Sometimes it represents treasure, and sometimes it represents remaining childhood. Regarding the inclination of the icons, there is a sense that insensitive to instability. In some cases, there may be an unexpected easy consciousness. | Want to enrich. Want to be satisfied. Abundant. |
| 6 | **10** | | 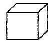 | If it is single, it represents a strong presence, and contact with other icons (7/14) is often simply a presence. Sometimes, contact with a line of the check sheet may represents a strong sense of stability and mental instability. If it is selected, it has a somewhat unique aspect. | Stable. Don't move. Want to enter in a box. Box. |

| D | Rounded-corner system | | | | |
|---|---|---|---|---|---|
| 7 | **8** | | 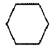 | Although there are little deep meanings, contact with other icons has many possibilities,and complex assessments are required when evaluating. If it is selected, it has a somewhat unique aspect. | Someone interested. A scary person. |
| 8 | **3** | | 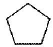 | If it is selected, it has a fairly unique aspect. In general, it is rarely selected, and it is difficult to use in terms of the overall configuration. It does not mean much if it is used as a human body. | Medicine. Have a dependence. Want to rely on. Want to calm down. |

**[TABLE 3B]**

| E | | Square system | | | |
|---|---|---|---|---|---|
| 9 | **2** | | 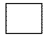 | Contact with the lines and corners of the check sheet is desirable to be strong stability and sense of security, and does not require a cluttered atmosphere. They often prefer organizing and do not prefer irresponsible things, but sometimes they do not. If it is used as the center of a house (contact with 7), not only is it a building, but also it is conscious of it as a house, and it needs to be paid attention. | Same as normal. |
| 10 | **6** | | 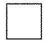 | Contact with the lines or corners of the check sheet is desirable to be strong stability and sense of security, and does not require a cluttered atmosphere. They prefer organizing, and often do not prefer irresponsible things. If it is used as the center of a house (contact with 7), not only is it a building, but also it is conscious of it as a house, and it needs to be paid attention. Sometimes they prefer some uniqueness, and contact with arrow (9) needs to he noted. | Slightly different from normal. |

| F | | Rhombic system | | | |
|---|---|---|---|---|---|
| 11 | **14** | | 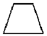 | Strong stabilization is desired, and dependence is often low. Reversal represents instability and may require request or help. In some cases, contact with (2) as a house has a different meaning and it is desirable to family cohesion. | Stable. Don't move easily. To be motivated. |
| 12 | **17** | | 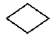 | Standard forms represents strong instability, but they may be insensitive, the instability and may not be problematic. Usually, it is applied in an inclined manner in many cases, and it represents stability. Contact with the icons (7) represents an inclined house and may represents instability within the family. Strong self-assertions are few, dependence may be strong, and it need to be satisfied. | Picky. Unstable Don't want to calm down. |

| G | | Sharp system | | | |
|---|---|---|---|---|---|
| 13 | **7** | | 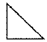 | The arrangement in the opposite Δ at 45 degrees slope represents a strong sense of instability and may indicate an unrest of mind. In many cases, two-sided contact at the four corners of the check sheet has a strong dependence and is required to be stable and secure. Contact with icon (2) represents the house and it needs to be analyzed as a house or a family. | Dangerous. Tight. Sharp, dangerous. Doubtful. |
| 14 | **15** | | 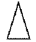 | It may represent sharpness and acuteness, and may represent a strong demand. It has a meaning as a sword and a meaning as a arrow, and it needs to be distinguished. In the case of a sword arrangement, it is necessary to interpret the meaning by paying attention to the orientation. In particular, contact with icons (11) needs to be paid attention. The same as for icon (1/5) | Cut. Dangerous. Stick. Be stuck. A cone. |

| H | | Unique system | | | |
|---|---|---|---|---|---|
| 15 | **9** | | 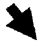 | The arrangement as a → is primarily , but it also has a mining as a spear. In particular, contact with icons (11) needs to be paid attention. The same as for icon (1/5). In addition, this may mean escape or release from a specific space, and the directionality in A1/A3/A4 needs to be noted. | Attention. See. Here. Perspective. |
| 16 | **12** | | 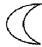 | It may be used as a moon simply, or it may be arranged in a structured manner by contact with icon (11). There is no concern if it is at the upper part of the check sheet. If it exists in A1/A3/A4, it may represent a strong sense of solitude. | Anxiety. Scared. Somehow. Vague. |
| 17 | **18** | | 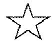 | If it exists at the upper part of the check sheet, it may indicate hope or pleasure, but if it exists at the bottom, it may indicate a sense of hopelessness and ask for help. If it is placed in A5, it has a self-assertion, and If it is placed in A1, it represents a strong sense of security. In the case of A3, it represents the existence as a treasure, and this is often the assertion that it is cherished. | Hope. Wish. Expectations. |
| 18 | **16** | | 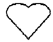 | Originally, it was influenced by color, but the color blue represents passive love and likes. If it exists in A1/A3/A4 itself, their own secret pleasures and hopes are existed. The incline of the icon may indicate distrust or discomfort, and it must be paid attention if it exists at the bottom of the check sheet. | Feeling. Like. Kind. |

**[TABLE 4]**

| | | | | | |
|---|---|---|---|---|---|
| | Total | | | It indicates the number of used icons, and if the number is large, it indicates positiveness or diversity. Conversely, if it is low, it indicates passiveness or prudence. If icons are used in excess of the standard, attention should be paid because of inattentiveness, strength of self-assertion, etc. If it is less than standard, it tends to be self-denial and to be hidden, it is difficult to communicate. | |
| **α** | **A system** | | | System A is the number of icons when icons are applied to locations other than lines (lines/frames) of the check sheet. This means that the line component of the check sheet is not taken into account. | |
| **β** | **○ system** | | | The system 0 is obtained by subtracting the system A from Total, and when it is 0, it indicates that icons do not exist on the line components (lines and frames) of the check sheet. There is also a sense of prudence and inability to overtake the line, but it is not be passive. | |
| | Total Number of Icons | | | Specified comment | |
| C o u n t | 2-5 | | Few | I don't want to know about myself. I want to hide. It's bothering. Attention deficit, decreased consciousness | |
| | 6-10 | | Ordinary | There is no protrusion. It is weak to restriction. Common. | |
| | 11-17 | | Many | Attention deficit, hot flashes, excessive consciousness, and frisk too mach. | |
| Icon overlapping and contact | | Overlapping | | There is an positiveness and breaking power. When the number is large, the degree of diversity is indicated, and the formed figure needs to be examined after evaluating the given meanings. | |
| | | Contact | | There is positiveness and breaking power, but at the same time there is prudence. When the number is large, there are diversity and spread of thought, and the formed figure needs to be examined after evaluating the given meanings. | |
| Line-contact | | 1st-contact | | In the case of contact with the lower frame, although there is a strong sense of stability and sense of security, there is a tendency to desire it. If it contacts with lines (lines or corners) other than the frame of the check sheet, it represents dependence, emphasis, and sense of security, and they are sought at the same time. If it contacts with the frame of the upper part, it may indicate anxiety or despondency. | |
| | | 2nd-contact | | If it contacts with any one of the four corners of lines of the check sheet, it indicates strong stability, security, and it is sought at the same time. Numbers represent its intensity When a line other than the frame is contacted, it prefers surprise, and there is a self-assertion different from the others. The search must be tested including contact between the icons. | |
| | Icon No. 11/ | | 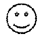 | It indicates the condition of the person (the person in question / the person) depending on the location of the existence (details are described separately) | |
| | Icon No. 18/ | | 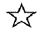 | It indicates the state of desire depending on the location of the existence (details are described separately) | |
| | Icon No. 12/ | | 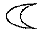 | It indicates the state of consciousness depending on the location of the existence (details are described separately) | |
| | Icon No. 16/ | | 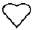 | It indicates the state of feeling depending on the location of the existence (details are described separately) | |
| | Icon No. 9/ | | 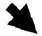 | It indicates the state in which the direction is placed depending on the location of the existence (details are described separately) | |
| | Icon No. 11/12 | | 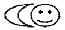 | It indicates the state of the symbolized person (the person in question / the person) depending on the location of the existence (details are described separately) | |
| | Icon No. 11/15 | | 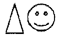 | It indicates the state of the person (the person in question / the person) who has a strong relationship with others depending on the location of the existence (details are described separately) | |

**[TABLE 5]**

| | Specific Pattern | Definitions | Specified Comment |
|---|---|---|---|
| Specific Situation | Crowded arrangement | A state that large number of icons clustered around a specific area | Strong dependence. Strong picky. Aggressiveness. Deflective. Envy jealousy. Cohesion. Can't do something irresponsibly. |
| | Dispersive arrangement | A state that icons are arranged in distributing in all or part of the area | Scatter. Distraction of consciousness. Can't break out of my shell. Unknown anxiety. Can't depend on someone. |
| | Balanced arrangement | A state that icons are arranged in a well-balanced manner in areas or line components | Equal Awareness, Averaging Awareness. Balance sensation, removing anxiety. Anxiety dispersion. Awareness of fragmentation. |
| | Deviative arrangement | A state in which icons are arranged on specific sides (right and left) or parts (top and bottom), including top, bottom, left, and right of the check sheet. | Diffuse feeling. Doing as I like. Slack. Deviation of consciousness. Indecision. |
| | Bottom arrangement | A state in which icons are arranged in close proximity or close proximity to the bottom of the check sheet (unbalanced state) | Stability orientation. Dependence. Seek peace of mind. Have confidence. Strong awareness of the current situation. No change. |
| | Protruding arrangement | A state that one or more of the icons protrude out of the check sheet frame (stepping on a line) | Excessive consciousness, irresponsible. Hot flashes. Elimination of Subjects. Stray. Confusing. |
| | Upper arangement | A state that most icons are arranged at the upper art (unbalanced state) | Unstable consciousness. To be motivated. Hope. Desire. First... First-time.. . |
| | Doll arrangement | A state that Tendency to form a Doll by arranging icons is seen | Human consciousness, targets. The person, this person, that person. Doll. Keepsake. Attack target Virtual-Object. |
| | Left-and-right Arrangement | A state that most icons are arranged on the left and right sides (unbalanced state) | Unstable. Segregation awareness. Escape from the center. Fear of the center. |
| | Top-and-bottom arrangement | A state that most icons are arranged at the top part and bottom (unbalanced state) | Anxiety. Somehow frightened. Unknown anxiety. |

**[TABLE 6]**

| | | Specific Pattern | Specified Comment |
|---|---|---|---|
| Interpretation of Specific Situation On (present) /off (none) | | Overlapping | Behavior under strong conscious. Abnormal. Consciousness expression. Biased thought. Excessive consciousness. Ignore rules. Egocentric. Integration consciousness. |
| | | Contact | Feelings to approach of interest. Awareness of interval. Dependence. Look into empathy. Want a dialogue or relationship. |
| | | Line-contact | Strong dependence. Want stability. Escape from loneliness. Consciousness according to rules. Telepathy. |

**[TABLE 7]**

| Number | Designation | Item | Definitions | Sample |
|---|---|---|---|---|
| 1 | A | Number | Number of applied icons (including protruding icons) | |
| 2 | B | 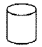 | If this icon is present, "1" is input. | |
| 3 | | 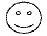 | If this icon is present, "1" is input. | |
| 4 | | 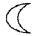 | If this icon is present, "1" is input. | |
| 5 | | 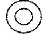 | If this icon is present, "1" is input. | |
| 6 | | 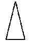 | If this icon is present, "1" is input. | |
| 7 | | 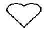 | If this icon is present, "1" is input. | |
| 8 | C | Crowded Arrangement | A case where many icons are gathered (in contact with each other or overlapped) in some areas | See Fig. 18A |
| 9 | | Dispersive Arrangement | A case where icons are applied mostly distributed | See Fig. 18B |
| 10 | | Balanced Arrangement | A case where they are applied one by one, for example, in each area in a balanced manner | See Fig. 18C |
| 11 | | Deviative Arrangement | A case where the icons are applied in an overlapping manner so as to form a cluster. | See Fig. 18D |
| 12 | | Bottom Arrangement | A case where three or more icons are applied in the bottom area (including the bottom line) | See Fig. 18E |
| 13 | | Upper Arrangement | A case where three or more icons are applied in the upper area | See Fig. 18F |
| 14 | | Top-and-Bottom Arrangement | A case where icons are applied to the top and bottom areas. with no icons in the middle area (other than the top and bottom areas) | See Fig. 18G |
| 15 | | Left-and-Right Arrangement | Acase where icons are applied to the right and/or left side areas, with no icons in the middle area (other than the left and right side areas) | See Fig. 18H |
| 16 | D | Protruding Arrangement | A case where any icon extends beyond the line of the frame, including a case where there is any icon stepping on the line of the frame | See Fig. 18I |
| 17 | E | Doll Arrangement | A case where the face and circular icons are applied to specified positions (A4/A5), and another case where they are applied to form a doll (a head and a body) at other positions | See Fig. 18J |
| 18 | F | Overlapping | A case where icons are given in an overlapping manner | See Fig. 18K |
| 19 | | Contact | A case where icons are in line-, face- or point-contact with each other | See Fig. 18L |
| 20 | | Close-Vicinity | A case where three or more icons are applied in close vicinity, nearly in contact with each other | See Fig. 18M |

**[TABLE 8]**

| table | TABLES and Figures | Major Contents |
|---|---|---|
| table 1 | TABLE 1A | Definition of lines |
| | TABLE 1B | Definition of corners |
| | TABLE 1C | Definition of areas |
| | TABLE 1D | Definition of contact points |
| table 2 | TABLE 2 | Definitions of types of the icons |
| | TABLE 3 | Icons' meanings |
| | TABLE 4 | Meaning of the total number of icons |
| | TABLE 7 | Overlapping and contact, etc. |
| table 3 | Fig. 3A to Fig. 3L | Given meaning depending on the position of a particular icon in the check sheet |
| table 4 | Fig. 4A to Fig. 4D | Given meaning depending on the positional relationship between the icons or between the icon and the line (icon relationship) |
| table 5 | TABLE 5 | Definitions of specific situation and specific patterns (10 types) |
| | TABLE 7 | |
| | Fig. 9A to Fig. 9H | |
| | TABLE 6 | Definitions of specific situation, overlapping, "contact, and line-contact |
| table 6 | TABLE 7 | Given meaning by appearance of a specific icon (doll) |
| | Fig. 9H. | |
| | Fig. 10. | |

Icons are selected and applied to the check sheet shown in Fig. 1 by the examinee. Icons identical to the icons shown in Fig. 1 are stored in a computer, not shown. In the present invention, the number of application of the icons is limited to 10-15.

The reason for this is that, in the present invention, it is necessary to test a large number of examinees rather than looking an individual examinee, and therefore, the number of application should be limited. Therefore, in the present invention, the icon size is reduced to the extent that the examinee would not feel frustration even when selecting the lowest number of the icons. In other words, in the present invention, in order to in order to occupy small space of icons in the check sheet even after the icons are applied to the check sheet so that the examinee may select and apply the icons with a satisfiable degree of freedom, the icons should be given in small sizes. This will expand the spatial recognition when the icons are applied to the check sheet.

In the storage means (not shown) of the computer, the definitions of the lines, areas, corners and contact points described in the check sheet are stored in advance in table 1, and the types of the icons and the definitions of their meanings are stored in table 2.

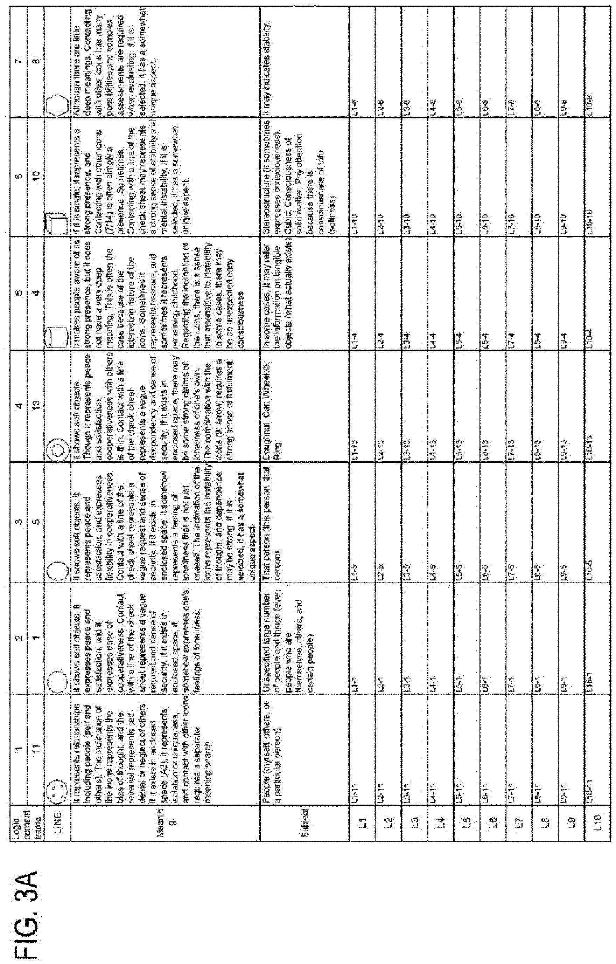

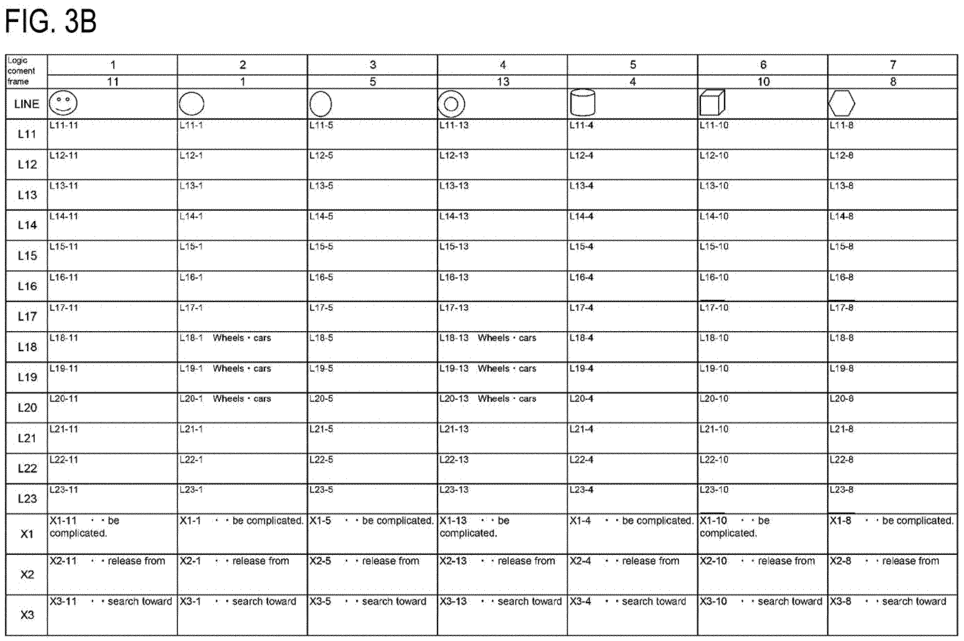

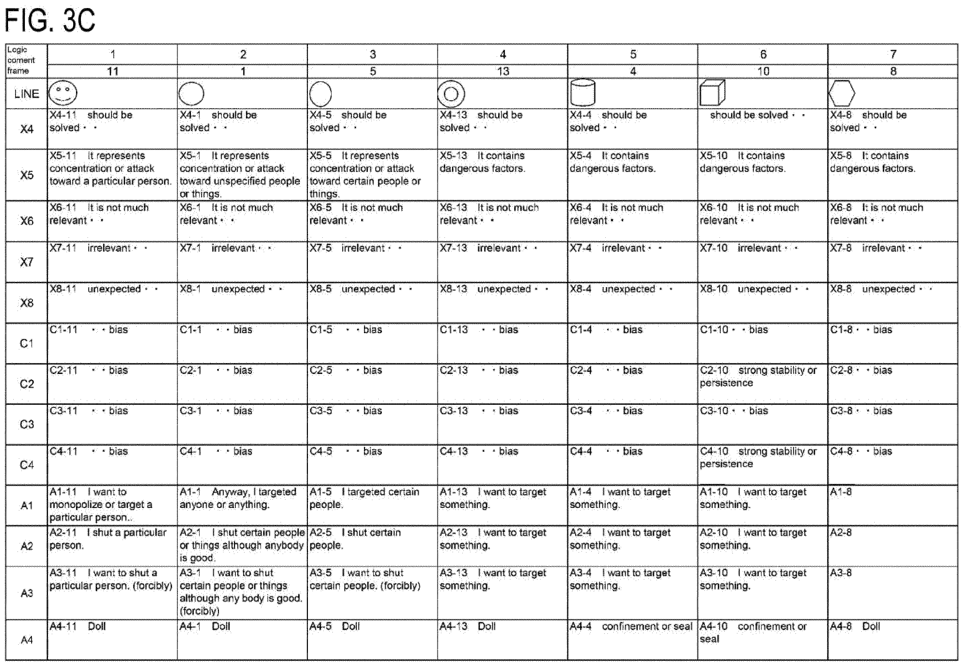

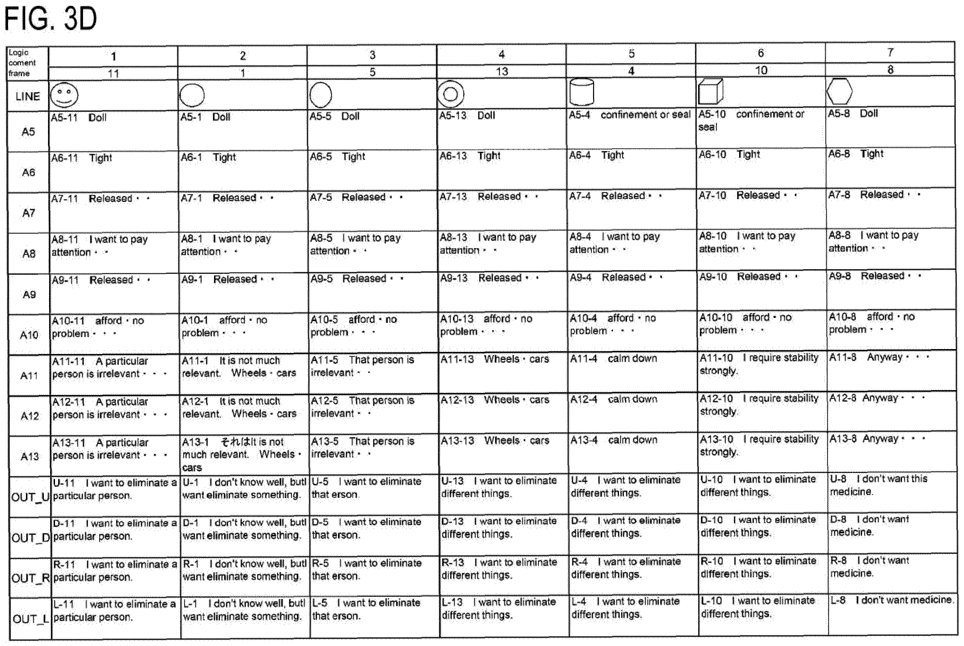

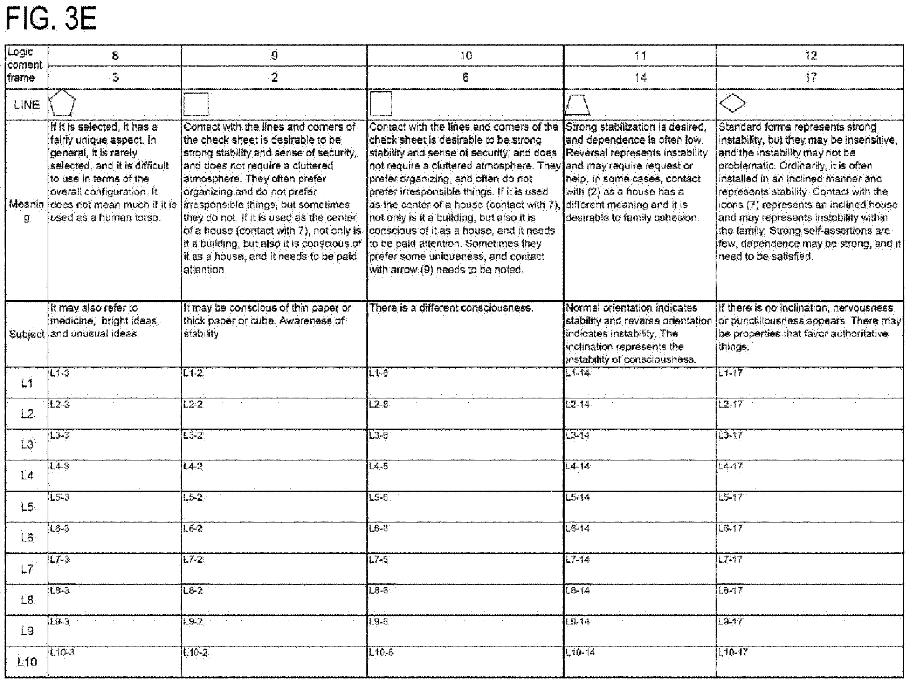

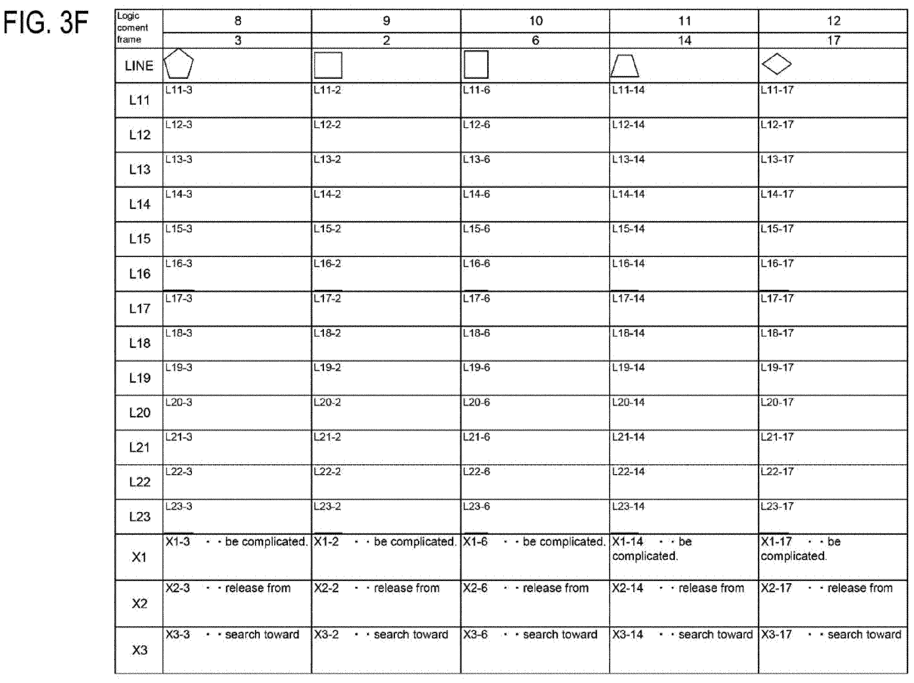

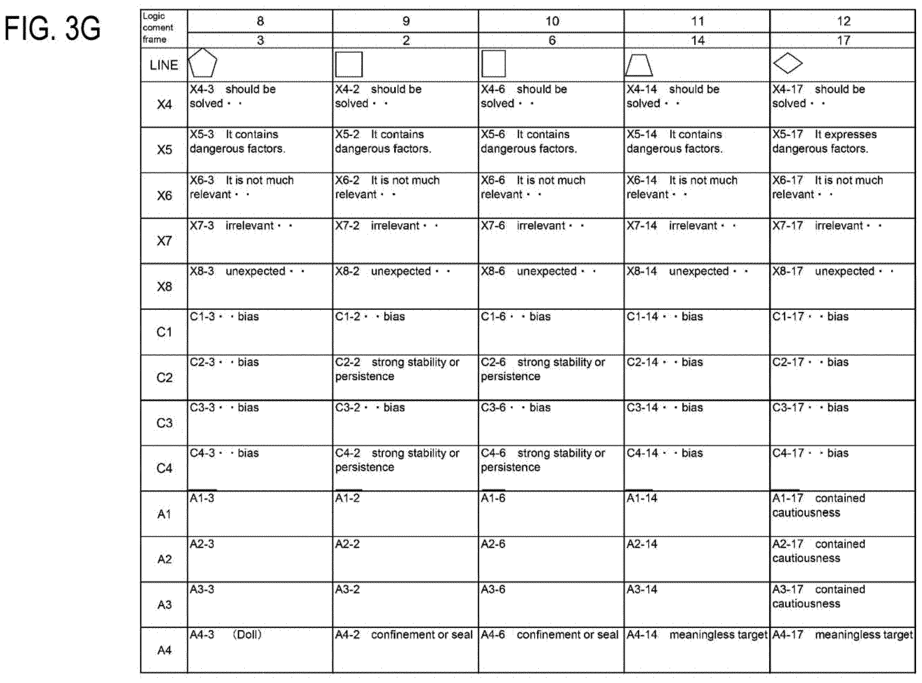

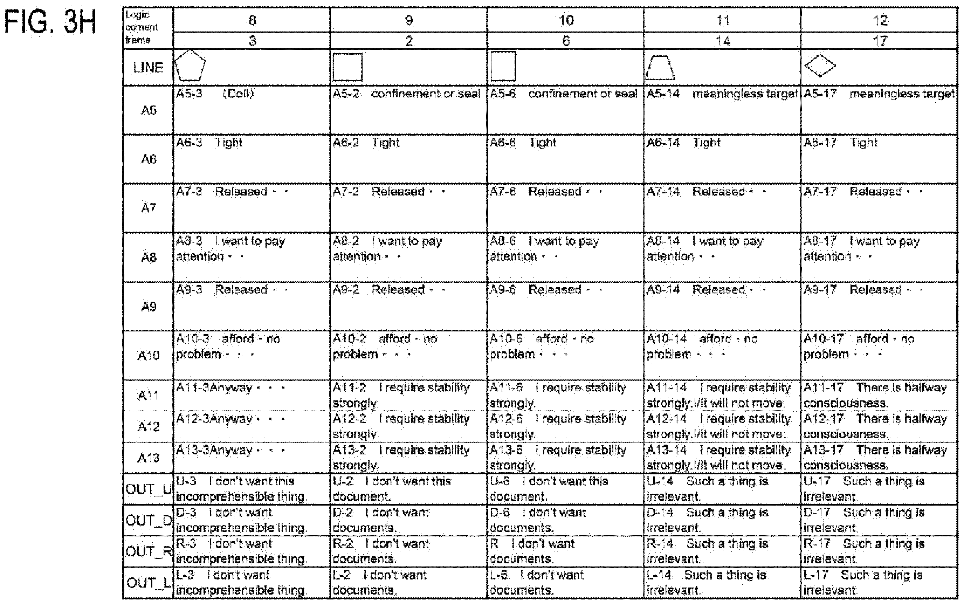

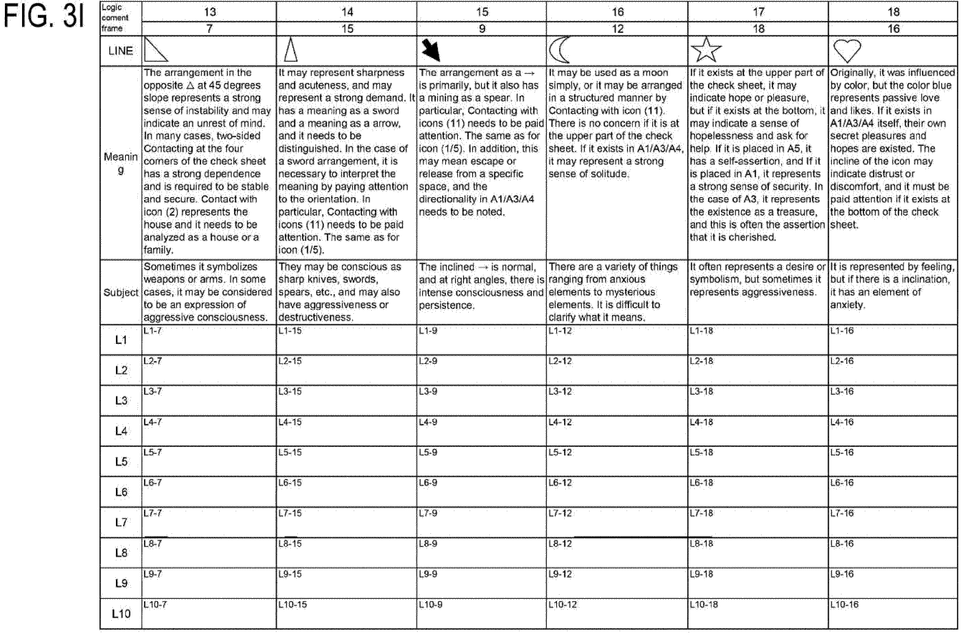

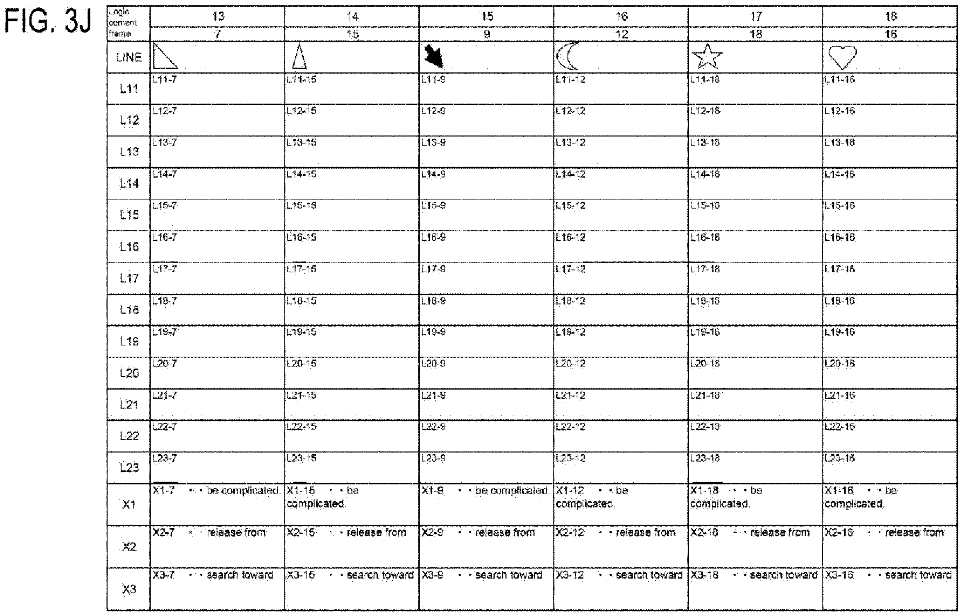

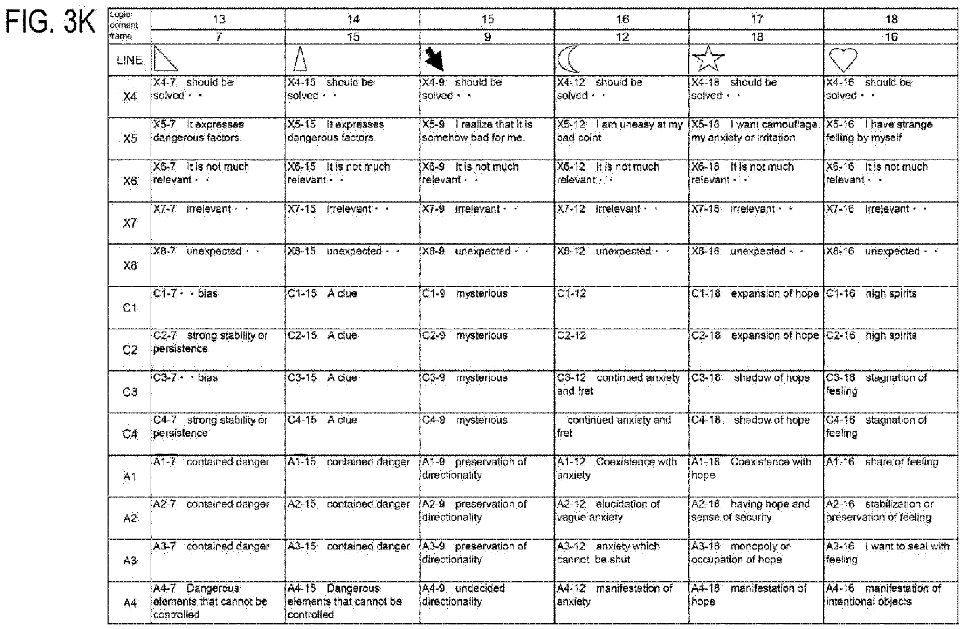

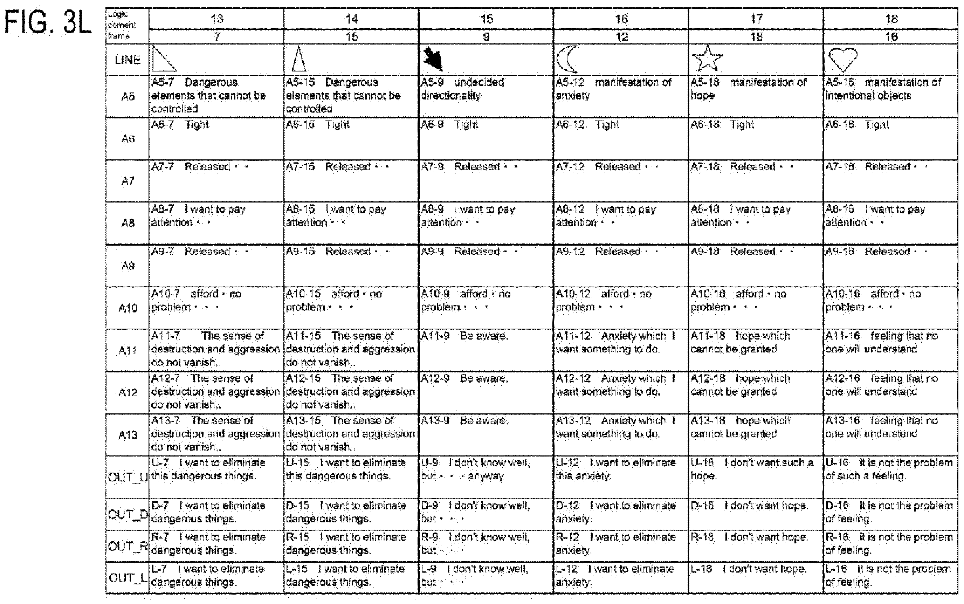

In table 3 in the storage means mentioned above, the given meaning definitions and code numbers as shown in Figs. 3A to 3L are stored depending on the position of a particular icon in the check sheet, i.e., on a particular line, at a particular corner, in a particular area or at a particular contact point.

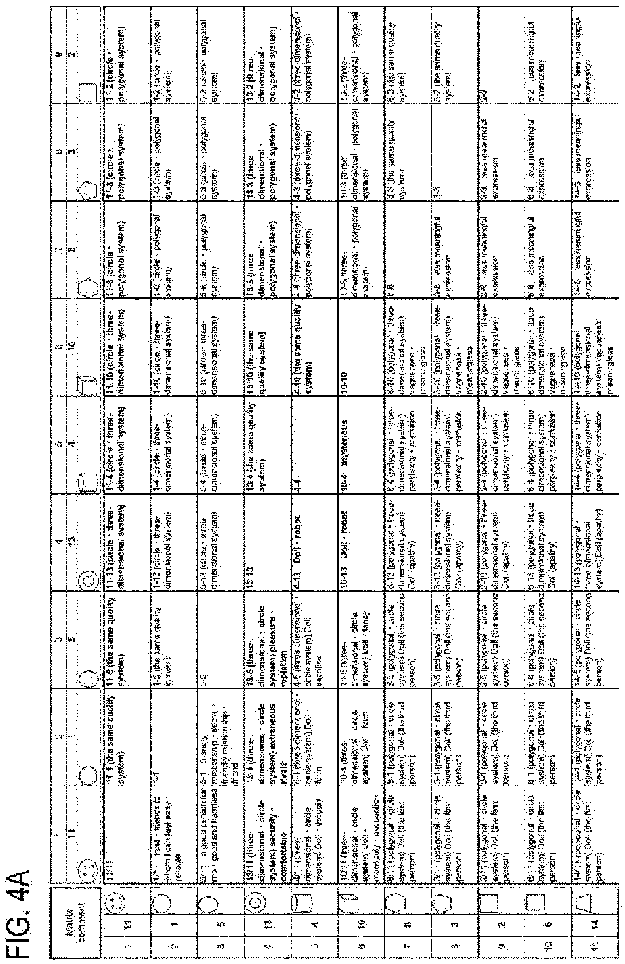

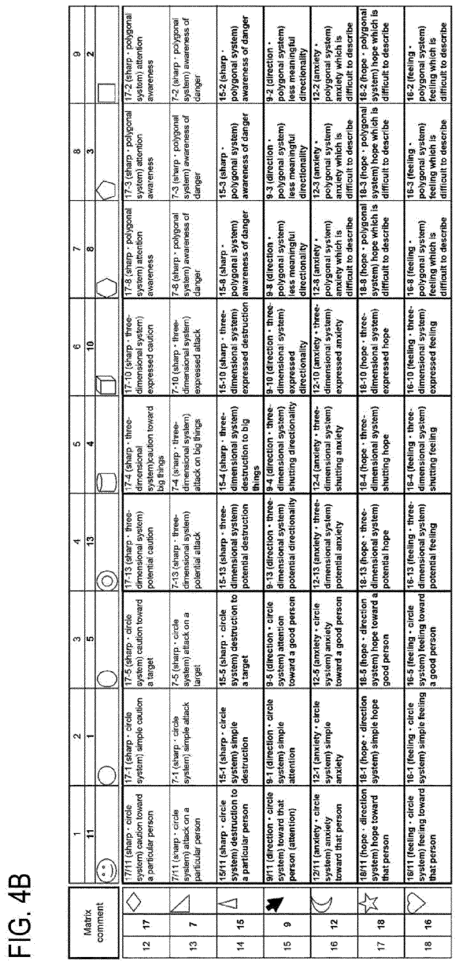

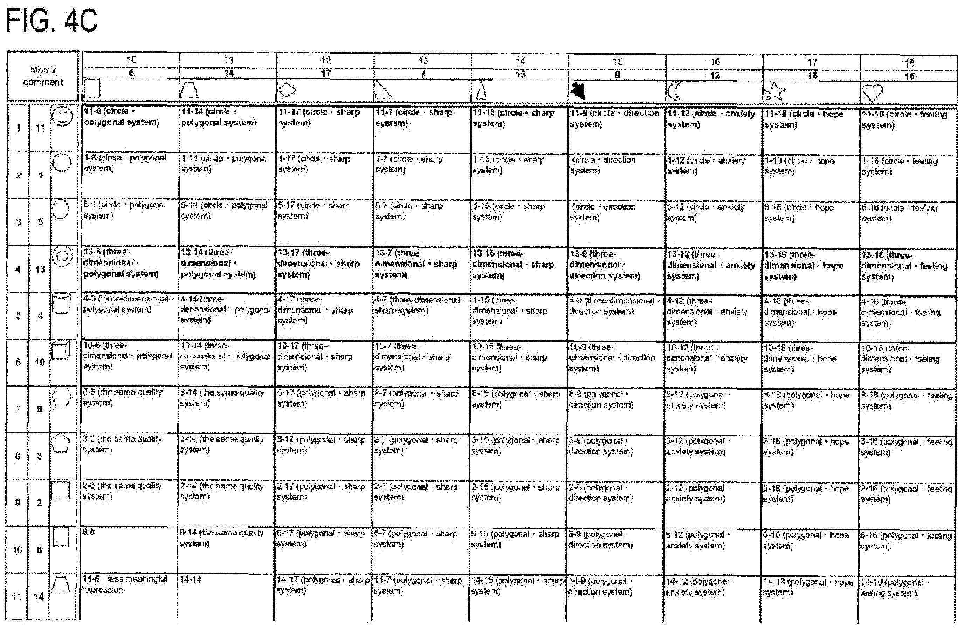

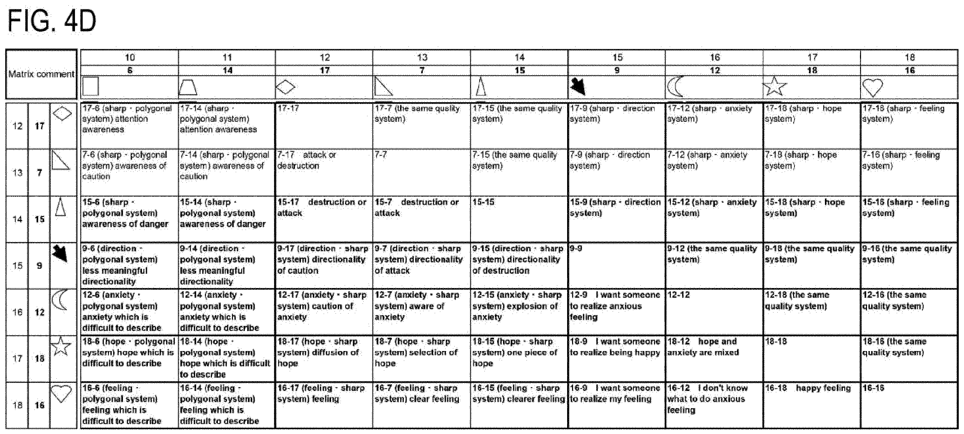

In the table 4 in the storage means mentioned above, the given meaning definitions and code numbers are stored as shown in Figs. 4A to 4D depending on the positional relationship between the icons or between the icon and the line (icon relationship).

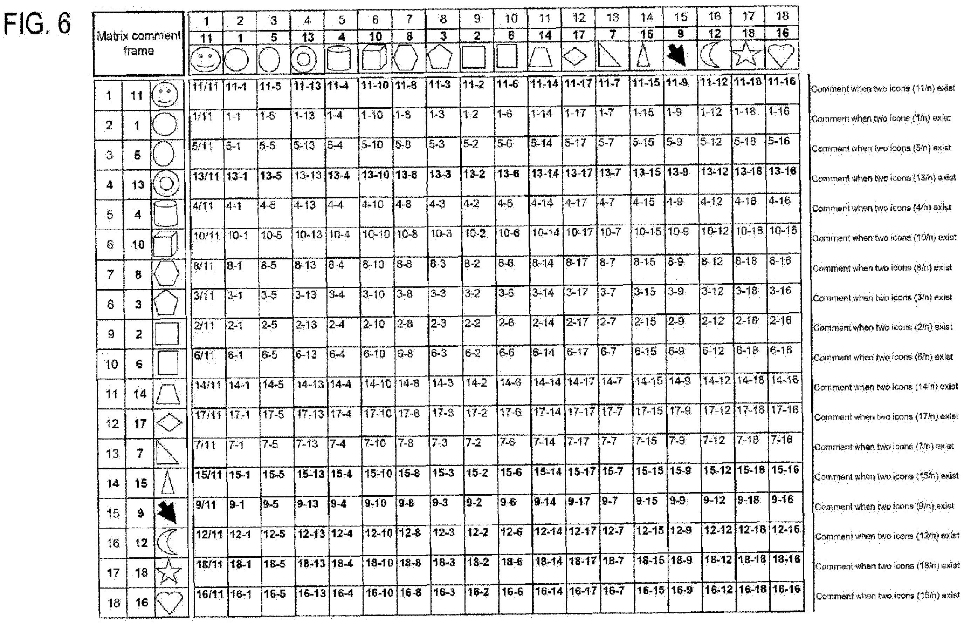

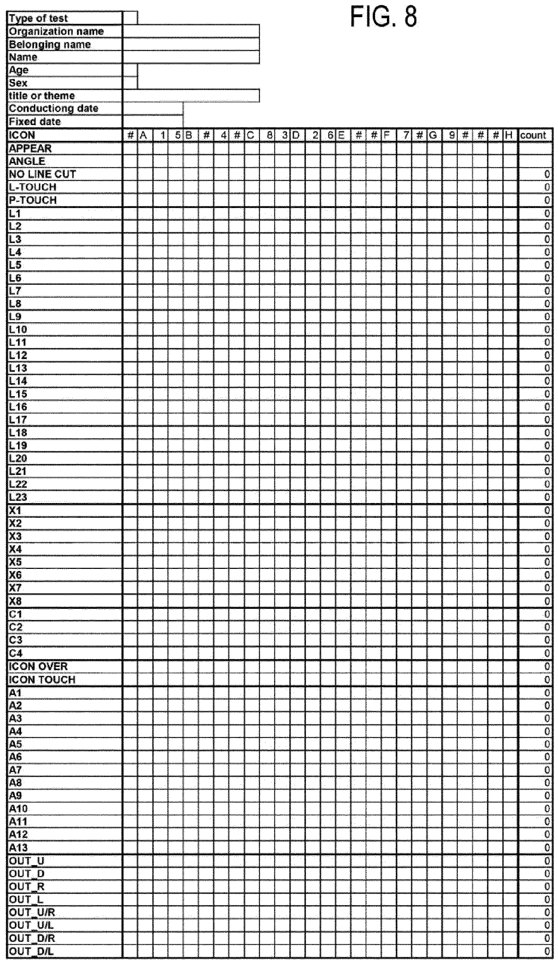

Figs. 5A to 5C are code tables (single) of the logic comments in table 3, and Fig. 6 is a code table (combination) of the logic comments in table 3. In addition, Figs. 7A to 7C are master sheets for test analysis, and Fig. 8 is a master sheet for the headquarter computer.

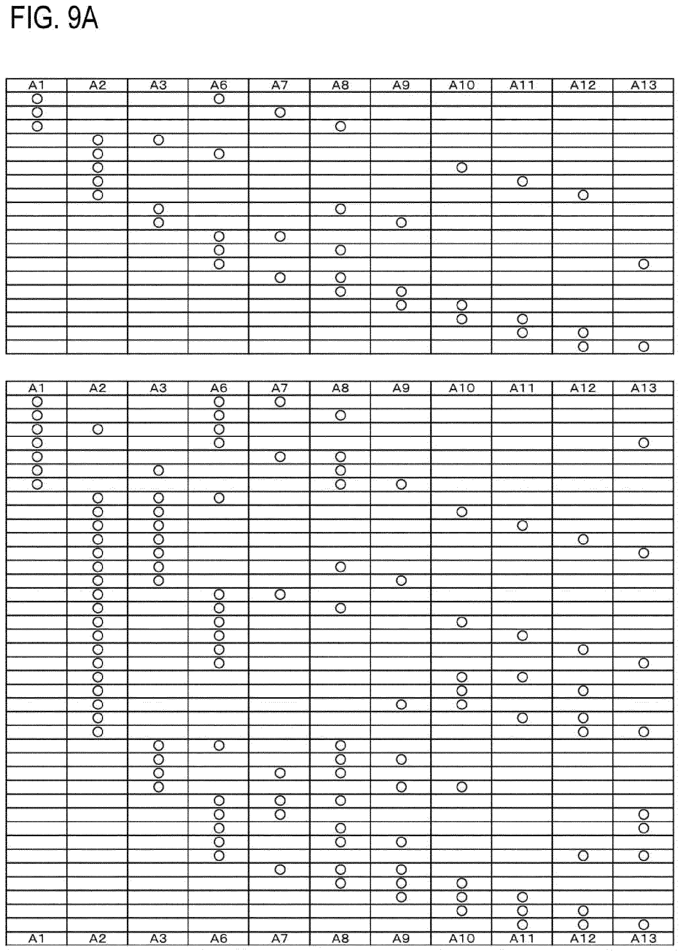

In table 5 in the storage means mentioned above, the specific patterns defined in TABLE 5 and TABLE 7 are stored as specific situation.
Ten types of the specific patterns will be described later with reference to Figs. 9A to 9G. In addition, in table 5 in the storage means mentioned above stores specific pattern defined for "overlapping", "contact", and "line-contact" shown in TABLE 6, as well as a "close-vicinity" to be described later.

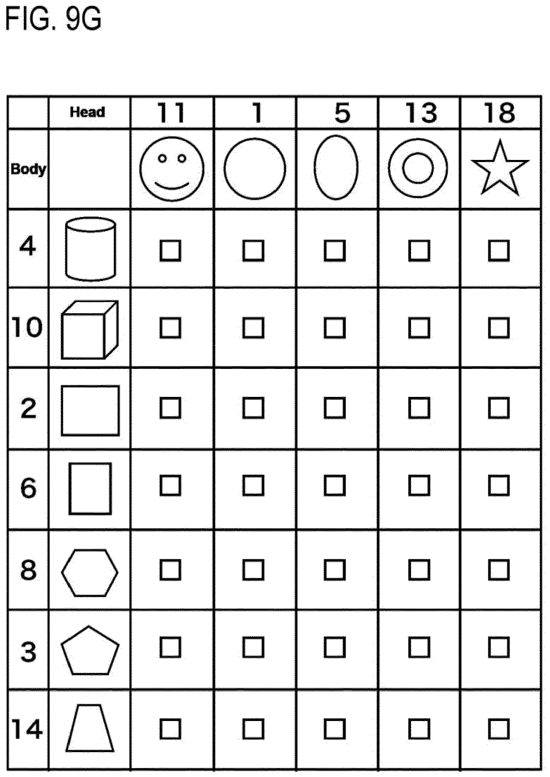

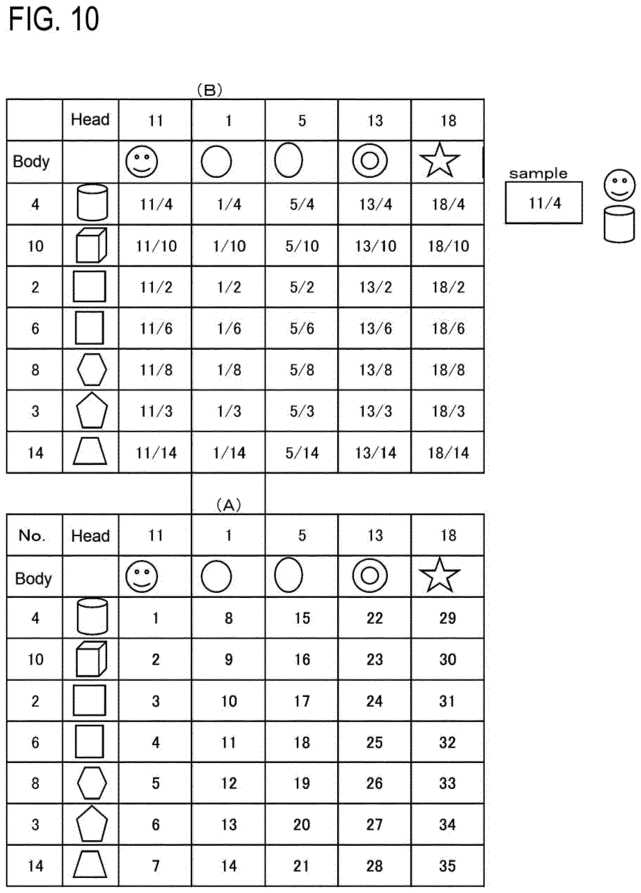

In table 6 in the storage means mentioned above, the definitions defined in advance as shown in TABLE 7, Fig. 9G and Fig. 10 with respect to the given meaning in a case wherein a specific icon appears.

Each icon described in TABLE 3A, TABLE 3B, and TABLE 4 are sorted into eight groups as follows and have designated comments stored in the above-described tables.

### A system (human system): Face

Want to be related to people. Have someone who I want to be targeted. Cannot ignore people. Have someone important. Have someone dislike.

### B system (circular system): circle, ellipse

Circle: kindness. Warmness. Calm. I'm not be nervous. Gentle.

Ellipse: soft. Good person. People (humans). Eggs, lukewarm. Can feel easy.

### C system (three-dimensional system): doughnut, column, cube

Doughnut: Strange feeling. With holes. Want to escape. Want to hide. Move. Car. Run.

Column: Want to enrich. Want to be satisfied. Abundant.

Cube: Stable. Don't move. Want to enter in a box. Box.

### D system (rounded-corner system): regular hexagon, regular pentagon

Regular hexagon: Someone interested. A scary person.

Regular pentagon: Medicine. Have a dependence. Want to rely on. Want to calm down.

### E system (square system): rectangle, square

Rectangle: Same as normal.

Square: slightly different from normal.

### F system (rhomboid system): trapezoid, rhomboid

Trapezoid: Stable. Don't move easily. To be motivated.

Rhomboid: Picky. Unstable. Don't want to calm down.

### G system (Sharp system): right triangle, isosceles triangle

Right triangle: dangerous. Tight. Sharp, dangerous. Doubtful.

Isosceles triangle: Cut. Dangerous. Stick. Be stuck. A cone.

### H system (unique system): arrow, moon, star, heart shape

Arrow: Attention. See. Here. Perspective.

Moon: Anxiety. Scared. Somehow. Vague.

Star: Hope. Wish. Expectations.

Heart shape: Feeling. like. Kind.

In addition, "specific situation" shown in TABLE 5 defines the following ten types of specific pattern in this embodiment.
Next, description will be made.

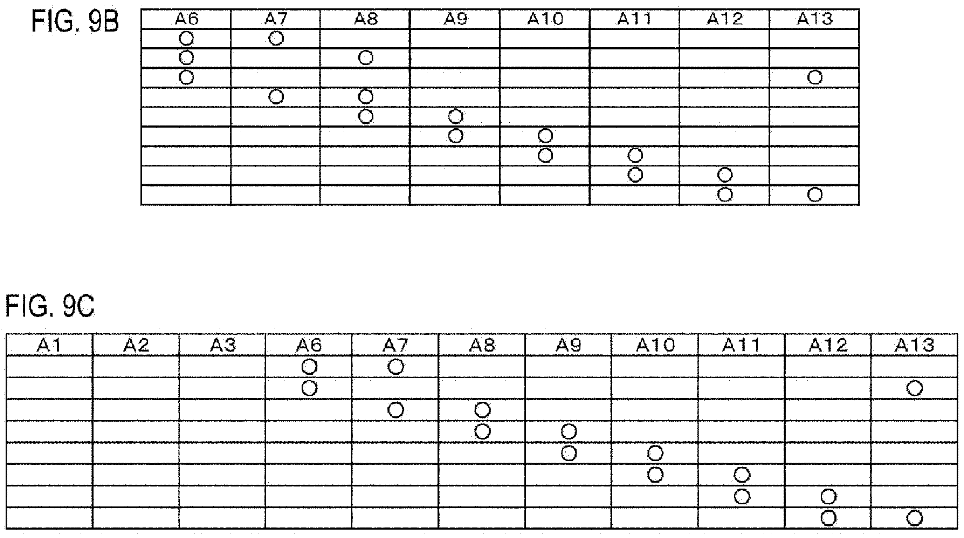

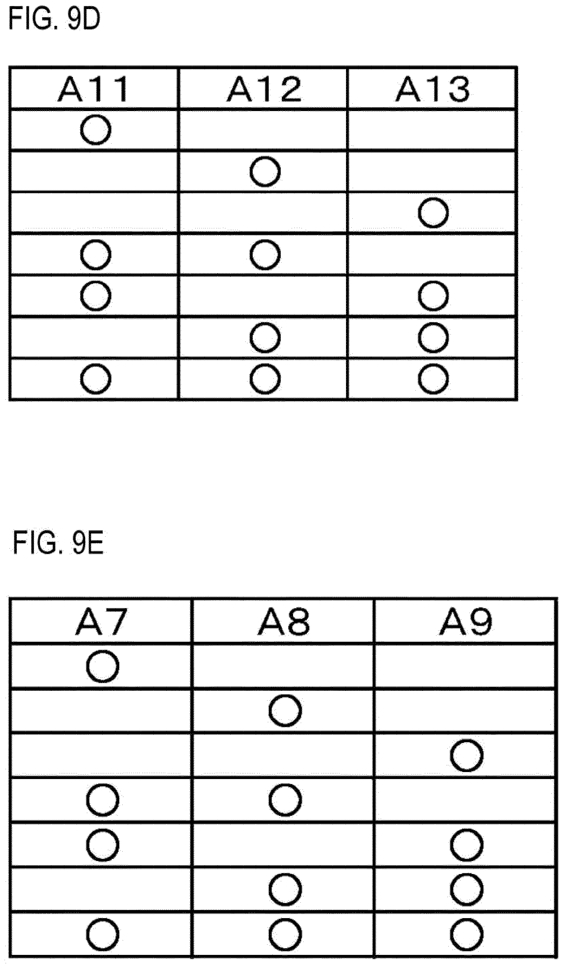

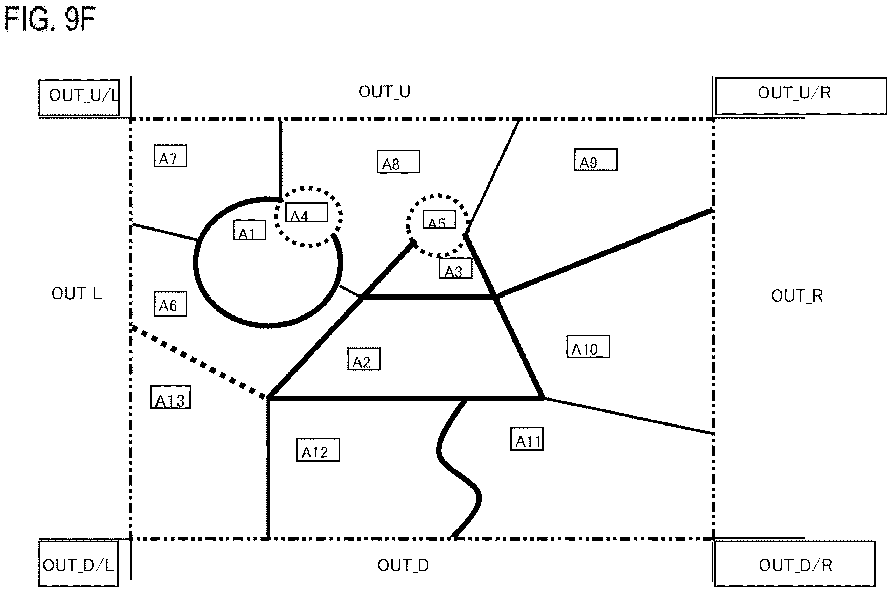

Figs. 9A to 9G are the specifications for the specific situation in table 5, in which Fig. 9A shows the calculation logic of "crowded arrangement", Fig. 9B shows the calculation logic of the " dispersive arrangement", Fig. 9C shows the calculation logic of the "deviative arrangement", Fig. 9D shows the calculation logic of the "bottom arrangement", Fig. 9E shows the calculation logic of the "upper arrangement", Fig. 9F shows the calculation logic of the "protruding arrangement", and Fig. 9G shows the calculation logic of the "doll arrangement".

The definitions and calculation logic of the specific situation are shown as follows.

The crowded arrangement refers to a case where a centroid of all the icons is located in two or three proximate areas. For example, it is in the areas A2, A10 and A9.

The calculation logic is as follows.
(A) The area A4 is determined as a part of either the area A1 or the area A8. In addition, the area A5 is determined as a part of either the area A3 or the area A8.
(B) A case where there is any icon not belonging to the areas A1-A13 will not be regarded as the "crowded arrangement".

The proximate areas have following 58 combinations.

<1> A1 and A6, <2> A1 and A7, <3> A1 and A8, <4> A2 and A3, <5> A2 and A6, <6> A2 and A10, <7> A2 and A11, <8> A2 and A12, <9> A3 and A8 <10> A3 and A9, <11> A6 and A7, <12> A6 and A8, <13> A6 and A13, <14> A7 and A8, <15> A8 and A9, <16>A9 and A10, <17> A10 and A11 <18> A11 and A12 <19> A12 and A13, <20> A1, A6 and A7, <21> A1, A6 and A8, <22> A1, A2 and A6, <23> A1, A6 and A13, <24> A1, A7 and A8, <25> A1, A3 and A8, <26> A1, A8 and A9, <27> A2, A3 and A6, <28> A2, A3 and A10, <29> A2, A3 and A11, <30> A2, A3 and A12, <31> A2, A3 and A13, <32> A2, A3 and A8, <33> A2, A3 and A9, <34> A2, A6 and A7, <35> A2, A6 and A8, <36> A2, A6 and A10, <37> A2, A6 and A11, <38> A2, A6 and A12, <39> A2, A6 and A13, <40> A2, A10 and A11, <41> A2, A10 and A12, <42> A2, A9 and A10, <43> A2, A11 and A12, <44> A2, A12 and A13, <45> A3, A6 and A8, <46> A3, A8 and A9, <47> A3, A7 and A8, <48> A3, A9 and A10, <49> A6, A7 and A8, <50> A6, A7 and A13, <51> A6, A8 and A13, <52> A6, A8 and A9, <53> A6, A12 and A13, <54> A7, A8 and A9, <55> A8, A9 and A10, <56> A9, A10 and A11, <57> A10, A11 and A12, <58> A11, A12 and A13. (Fig. 9A).

The dispersive arrangement refers to a case where one or two icons are located in an area not in vicinity to the lines. In this case, areas A1, A2 and A3 are not targets of proximate lines.

The calculation logic is as follows.
(A) In a case where two or more icons exist in any one of areas A1, A2 and A3,
(B) In a case where there is any icon not belonging to areas A1-A13,
(C) A case where three or more icons exist in any one of areas A4 to A13 will not be regarded as the dispersive arrangement.
(D) It does not fall under the dispersive arrangement, if <1> there is at least one icon belonging to Area A6 and A7, <2> there is at least one icon in A6 and A8, <3> there is at least one icon in A6 and A13, <4> there is at least one icon in A7 and A8, <5> there is at least one icon in A8 and A9, <6> there is at least one icon in A9 and A10, <7> there is at least one icon in A10 and A11, <8> there is at least one icon in A11 and A12, <9> there is at least one icon in A12 and A13 (Fig. 9B).

The balanced arrangement refers to a case where there is only one icon in one area.

The calculation logic is as follows. The following cases will not be regarded as the "balanced arrangement".
<1> If there are icons not belonging to areas A1 to A13,
<2> If two or more icons exist in one area,
<3> If the icons exist which number is less or more than the prescribed numbers.

The deviative arrangement refers to a case where all icons exist in the outermost two areas. However, areas A4 and A5 should be considered as area A8.

The calculation logic is as follows. The following cases should be regarded as the deviative arrangement. (Fig. 9C)
<1> All icons exist in areas A6 and A7
<2> All icons exist in areas A6 and A13
<3> All icons exist in areas A7 and A8
<4> All icons exist in areas A8 and A9
<5> All icons exist in areas A9 and A10
<6> All icons exist in areas A10 and A11
<7> All icons exist in areas A11 and A12
<8> All icons exist in areas A12 and A13

The bottom arrangement refers to a case where all icons exist in the bottom areas A11, A12 and A13.

Therefore, the bottom arrangement includes the following seven cases by this calculation logic (Fig. 9D).
<1> area A11
<2> area A12
<3> area A13
<4> areas A11 and A12
<5> areas A11 and A13
<6> areas A12 and A13
<7> areas A11, A12 and A13

The upper arrangement refers to a case where all icons exist in the upper areas A7, A8 and A9.

Therefore, the upper arrangement includes the following seven cases by this calculation logic (Fig. 9E).
<1> area A7
<2> area A8
<3> area A9
<4> areas A7 and A8
<5> areas A7 and A9
<6> areas A8 and A9
<7> areas A7, A8 and A9

The protruding arrangement refers to a case where all icons are stepping on or protruding outward from any of the outer peripheral lines L-1 to L-7.

The calculation logic for the protruding arrangement is determined not based on the centroid of the ions but based on the area and line data from the analysis engines.

If the flags of the icons in the following eight areas shown in Fig. 9F are all ON, it is regarded as the "protruding arrangement".
<1> OUT U/L
<2> OUT U
<3> OUT U/R
<4> OUT R
<5> OUT D/R
<6> OUT D
<7> OUT D/L
<8> OUT L.

The top-and-bottom arrangement refers to a case where all icons exist in the upper areas A-7, A-8 and A-9 and in the lower areas A-13, A-12 and A-11, which is the calculation logic therefor.

The left-and-right arrangement refers to a case where all icons exist in the left areas A-7, A-6 and A-13 and in the right areas A-9, A-10 and A-11, which is the calculation logic therefor.

The doll arrangement refers to a case where some face icon exists in A4 and/or A5 to form a doll.

The calculation logic for the doll arrangement is as follows. First, the icons which may form a face include
Face (Icon No. 11),
Circle (Icon No. 1),
Ellipse (Icon No. 5),
Doughnut (Icon No. 13),
Star (Icon No. 18).

In addition, it is recognized as the doll arrangement if the body icon(s) is combined with the face icon in the following manners (Fig. 9G).
<1> If the face icon (Icon No. 11) is combined with column (Icon No. 4), cube (Icon No. 10), square (Icon No. 2), rectangle (Icon No. 6), regular hexagon (Icon No. 8), regular pentagon (Icon No. 3) or trapezoid (Icon No. 14)
<2> If the circle icon (Icon No. 1) combined with column (Icon No. 4), cube (Icon No. 10), square (Icon No. 2), rectangle (Icon No. 6), regular hexagon (Icon No. 8), regular pentagon (Icon No. 3) or trapezoid (Icon No. 14)
<3> If the ellipse icon (Icon No. 5) combined with column (Icon No. 4), cube (Icon No. 10), square (Icon No. 2), rectangle (Icon No. 6), regular hexagon (Icon No. 8), regular pentagon (Icon No. 3) or trapezoid (Icon No. 14)
<4> If the doughnut icon (Icon No. 13) combined with column (Icon No. 4), cube (Icon No. 10), square (Icon No. 2), rectangle (Icon No. 6), regular hexagon (Icon No. 8), regular pentagon (Icon No. 3) or trapezoid (Icon No. 14)
<5> If the star Icon (Icon No. 18) combined with column (Icon No. 4), cube (Icon No. 10), square (Icon No. 2), rectangle (Icon No. 6), regular hexagon (Icon No. 8), regular pentagon (Icon No. 3) or trapezoid (Icon No. 14)

Fig. 10 shows criteria separately provided for determining the details of the doll arrangement. This is also stored in the computer's storage means.

Next, "overlapping", "contact", "line-contact" and "close-vicinity" described in TABLE 6 will be described. The "overlapping" refers to a situation in which icons overlap each other, and "contact" refers to a situation in which the peripheral parts of the icons contact each other, including "line-contact" meaning a situation in which the peripheral part of the icon contact with the line in the check sheet. The "close-vicinity" refers to a situation in which the icons touch the line of the other icons and check sheets.

TABLE 7 is a template showing the meaning of the icons input to the computer. Based on TABLE 7, the presence or absence of an icon seal picked up and applied to the check sheet by the examinee is input. Incidentally, when the computer cannot determine the application state of the icons, it may be done by the person in charge according to TABLE 7.

The "designation" column A-F in TABLE 7 have the following meanings.
A: Number designation
B: Selection and designation of specific icon
C: Arrangement designation
D: Protruding designation
E: Doll designation
F: Overlapping, Contact, Close-Vicinity designation

Based on TABLE 7, if there is any icon applied on the check sheet, its icon number is input. For example, "1" is input if there is "column" in the designation column B, and "0" is input if there is not. The "crowded arrangement", "dispersive arrangement", "balanced arrangement", "deviative arrangement", "bottom arrangement", "upper arrangement", "top-and-bottom arrangement" and "left-and-right arrangement" in the designation column C is determined based on the typical examples shown by reference in Fig. 18A to Fig. 18M, and "1" is input if any, and "0" is input if none. If the computer is unable to make a determination, it may be done by the person in charge according to TABLE 7. Similarly, if the " protruding arrangement" is recognized in the designation column D, "1" is input, and "0" is input if it is not. In the designation column E, "1" is input if it is recognized as the "doll arrangement", and "0" is input if it is not. If the "overlapping", "contact" or "close-vicinity" is recognized in the designation column F, "1" is input, and "0" is input if it is not.

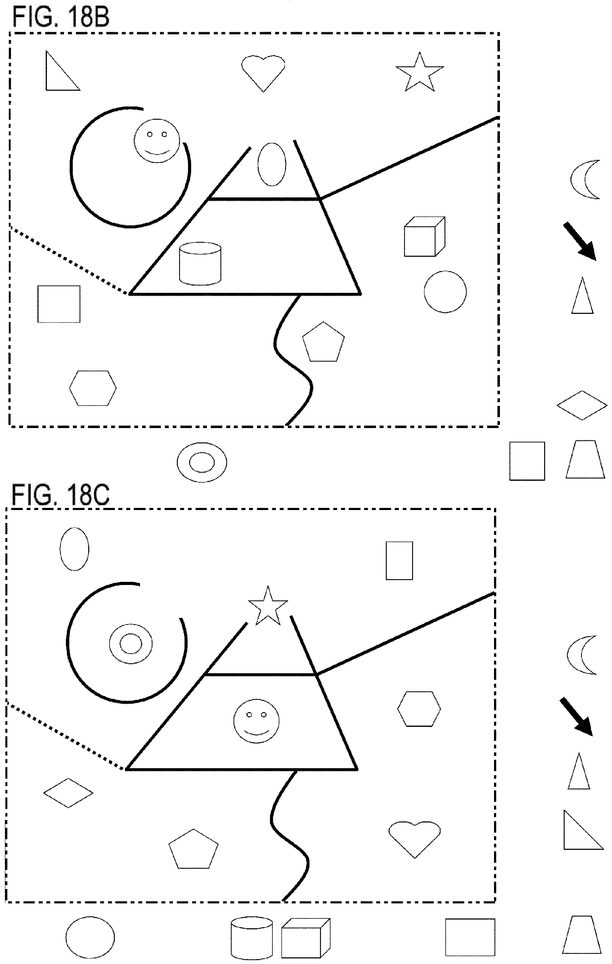

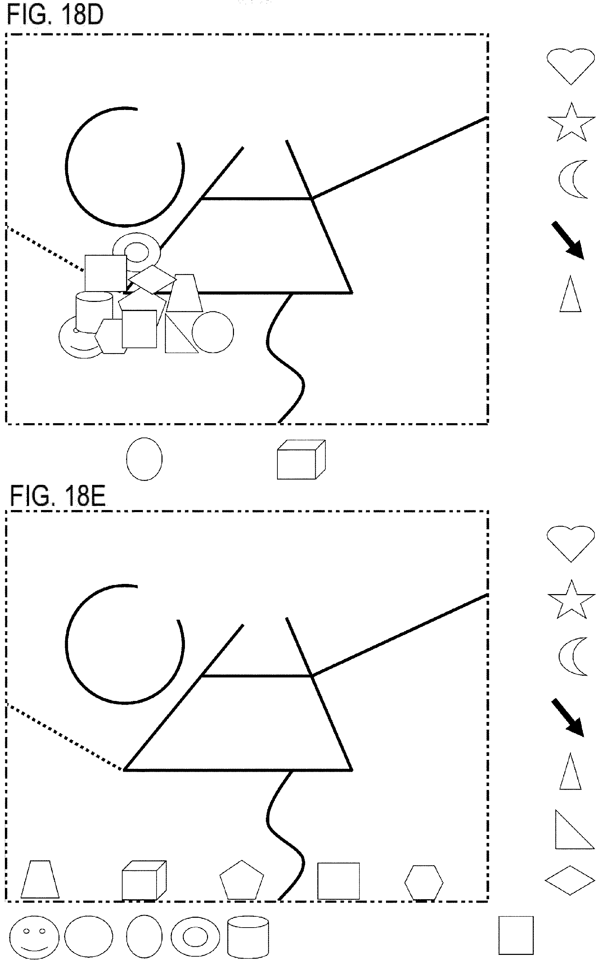

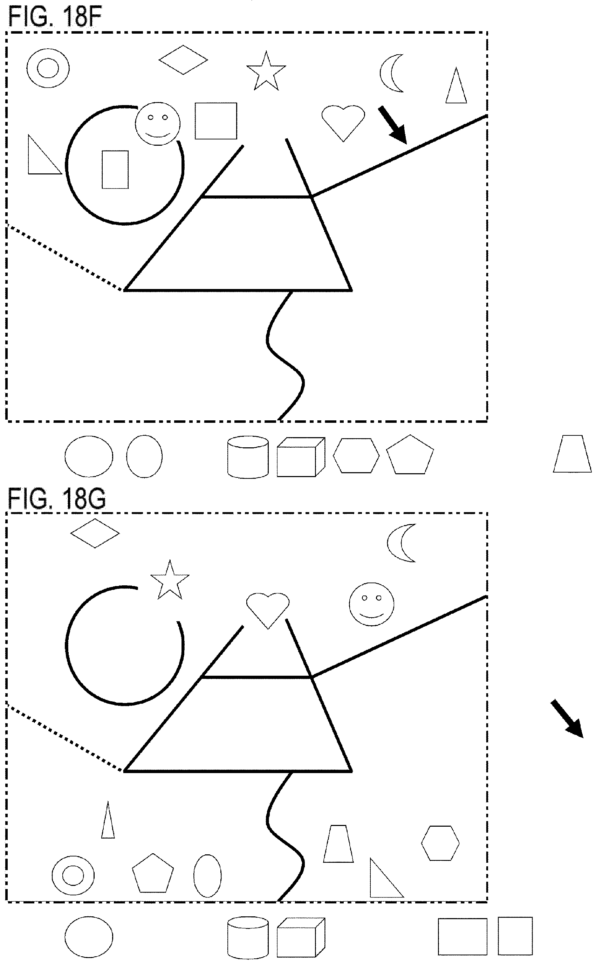

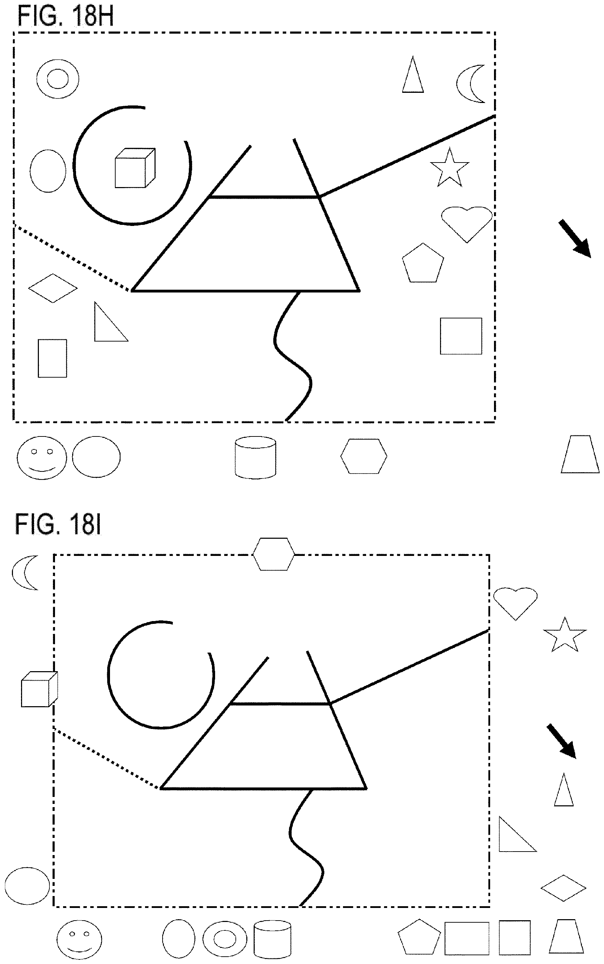

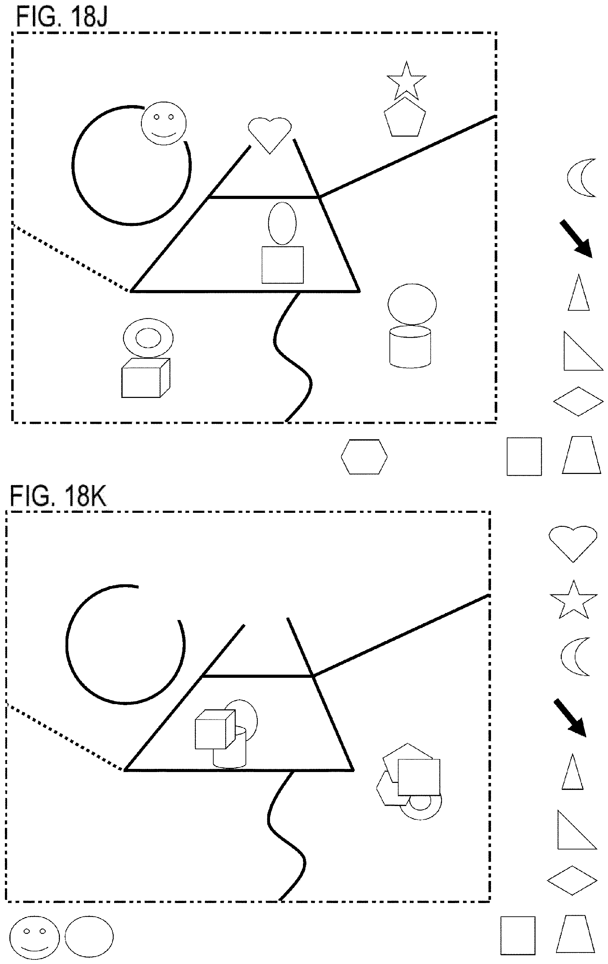

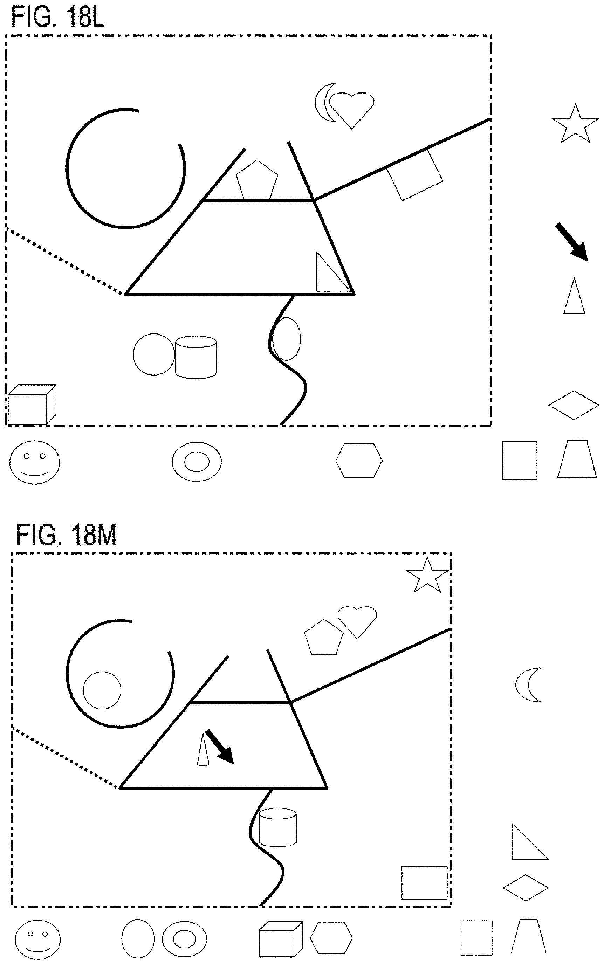

The definitions of the items in the columns C to F in TABLE 7 are as follows.
Crowded Arrangement: a case where many icons are gathered (in contact with each other or overlapped) in some areas (see the typical example in Fig. 18A)
Dispersive Arrangement: a case where icons are applied mostly distributed (see the typical example in Fig. 18B)
Balanced Arrangement: a case where they are applied one by one, for example, in each area in a balanced manner (see the typical example of Fig. 18C)
Deviative Arrangement: a case where the icons are applied in an overlapping manner so as to form a cluster (see the typical example in Fig. 18D)
Bottom Arrangement: a case where three or more icons are applied in the bottom area (including the bottom line) (see the typical example in Fig. 18E)
Upper Arrangement: a case where three or more icons are applied in the upper area (see the typical example in Fig. 18F)
Top-and-Bottom Arrangement: a case where icons are applied to the top and bottom areas, with no icons in the middle area (other than the top and bottom areas) (see the typical example in Fig. 18G)
Left-and-Right Arrangement: a case where icons are applied to the right and/or left side areas, with no icons in the middle area (other than the left and right side areas) (see the typical example in Figure 18H)
Protruding Arrangement: a case where any icon extends beyond the line of the virtual frame, including a case where there is any icon stepping on the line of the virtual frame (see the typical example in Fig. 18I)
Doll Arrangement: a case where the face and circular icons are applied to specified positions (A4/A5), and another case where they are applied to form a doll (a head and a body) at other positions (see the typical example in Fig. 18J).
Overlapping: a case where icons are given in an overlapping manner (see the typical example in Fig. 18K)
Contact: a case where icons are in line-, face- or point-contact with each other (see the typical example in Fig. 18L)
Close-Vicinity: a case where three or more icons are applied in close vicinity, nearly in contact with each other (see the typical example in Figure 18M)
Figs. 18A to 18M show typical examples described in the "Sample" column in TABLE 7.

In the designation field C, normally one item is select, but up to two items may still be selected. As a result, the arrangement state can be grasped first. However, with respect to the one representing the positions of the top, bottom, left and right, there is some combination which does not cause any inconsistency even in an overlapping manner, and therefore, the system is set up so as to allow such combination.

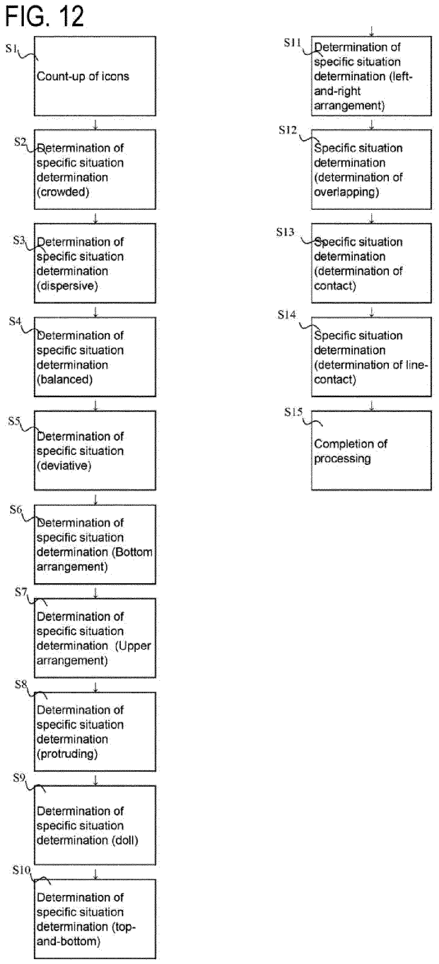

A computer having the above-described configuration is used to determine an examinee's mental structure. An examinee applies a predetermined number (10-15) of desired icons to desired positions on the check sheet within a time limit (about 5 minutes).
Based on the flow chart of Fig. 12, the check sheet to which the icons have been applied is processed by the computer in steps S1 to S15.

Next, a processing procedure by the computer will be described with reference to Fig. 12. First, the total number of the icons applied to the check sheet is counted ("Count Up" ▪ S1).

Specific situation of the icon application is then determined. The determination of the specific situation is determination as to whether or not the icon application corresponds to all or part of "Crowded arrangement" (S2), "Dispersive arrangement" (S3), "Balanced arrangement" (S4), "Deviative arrangement" (S5), "Bottom arrangement" (S6), " Upper arrangement" (S7), "Protruding arrangement" (S8), "Doll arrangement" (S9), "Top-and-Bottom arrangement" (S10), and "Left-and-Right arrangement" (S11). A series of determination steps of "specific situation" (S2 to S11) is a step of extracting the application state of the icons.

If "specific situation" is present (on), the determination is made as follows.

Crowded Arrangement: Strong dependence. Strong picky. Aggressive. Deflective. Envy jealousy. Density. Can't do something irresponsibly.

Dispersed Arrangement: scatter. Distraction of consciousness. Can't break out of my shell. Unknown anxiety. Can't depend on someone.

Balanced Arrangement: Equal Awareness, Averaging Awareness. Balance sensation, removing anxiety. Anxiety dispersion. Awareness of fragmentation.

Deviative Arrangement: Diffuse feeling. Doing as I like. Slack. Deviation of consciousness. indecision.

Bottom Arrangement: Stability orientation. Dependence. Seek peace of mind. Have confidence. Strong awareness of the current situation. No change.

Protruding Arrangement: Excessive consciousness, irresponsible. Hot flashes. Elimination of Subjects. Stray. confusing.

Upper Arrangement: Unstable consciousness. To be motivated. Hope. Desire. First... First-time... Virtual-Object.

Doll Arrangement: Human consciousness, targets. The person, this person, that person. Doll. Keepsake. Attack target

Left-and-Right Arrangement: unstable. Segregation awareness. Escape from the center. Fear of the center.

Top-and-Bottom Arrangement: Anxiety. Somehow frightened. Unknown Anxiety.

Next, the "overlapping" (S12), "contact" (S13) and "line-contact" (S14) are determined for the selected and applied icons. These steps determine the refractive directionality ("direction") of the examinee's personality. For example, when the amount of the "overlapping" is large, it is determined that he/her tends to have a strong deflecting property, and when the amount of the "line-contact" is large, it is determined that he/her tends to be stable. The details are as follows.

Overlapping: Behavior under strong conscious. Abnormal. Consciousness expression. Biased thought. Excessive consciousness. Ignore rules. Egocentric. Integration consciousness.

Contact: Feelings to approach of interest. Awareness of interval. Dependence. Look into empathy. Want a dialogue or relationship.

Line-contact: Strong dependence. Want to be stable. Escape from loneliness. Consciousness according to rules. Telepathy.

The determination of the doll arrangement is based on table 6 (Fig. 10) in which the definitions for formations of face and body are stored. With respect to this determination, the person in charge manually modifies the processing result based on the above-mentioned criteria, if necessary.

Thus, when the processing for an examinee is completed (S15), the process returns to Step 1, and the above-described series of steps is performed for the next examinee.

Next, the content of evaluation in each step will be described. The count-up evaluation of the total icon number is as follows.
<1> If the count-up number is not limited, evaluation is performed as follows.
   0: The response is evaluated as unresponsive or simply rejected (not want to respond).
   1∼17: It is evaluated as motivative, self-expressive and self-assertive (high).
   18 (ALL): It is evaluated as self-assertive (fairly high), much motivative, much interested.
<2> There may be a limit on the number of count-up (10-18)
   9 or less: It is evaluated as misunderstanding, little responsive or simply rejected (not want to respond).
   10-17 (Selected): It is evaluated as motivated, self-expressive and self-assertive (much or high).
   18 (ALL): It is evaluated as misunderstanding, self-assertive (fairly high), much motivated and much interested.

As described above, mental structure i.e. mental functions will include ones to be grasped as amygdala responses and others to be grasped as neocortex responses. A person responds to a stimulus by amygdala earlier than by neocortex by about 100ms. Amygdala's responses include likes and dislikes, perceptions of dangers, aversions and strange feeling, etc. Amygdala emits dangerous signals to an alien substance (including unknown substances) entering the body, and reflex nerve motion or the like is immediately performed without passing through neocortex.

On the other hand, the neocortex's response is recognized as being influenced by the experiences and knowledge of a certain individual. This means that what passed through the reaction of amygdala is ultimately determined by the neocortex's response.

The present invention is based on the recognition that selection and arrangement of the icons include one resulting from the amygdala's response and the other resulting from the neocortex's response, by which the analysis is performed.

In this regard, the overlapping, contact and line-contact is evaluated as follows in the specific situation interpretation steps.

The "overlapping" represents a condition in which a plurality of icons are overlapped, which may be regarded as a response by amygdala.
The selected and applied icons are recognized as representatives of people and/or goods, and the potential consciousness and tendency such as hiding and crushing are expressed by the overlapping.

The "contact" represents a condition in which the peripheral parts of the icons are in contact with each other, which may be regarded as a response by neocortex. This can be identified as a response representing a close relationship but not reaching the overlapping.

The "line-contact" represents a condition in which the lines in the check sheet and the peripheral parts of the icons are in contact with each other, which may be regarded as a response by amygdala. This is a representative one of the strange feelings but is only occasional. This can be grasped as an unconscious reaction not much given by neocortex.

The "close-vicinity" represents a condition in which an icon is in contact with another icon and/or the line components of the check sheet, which is a neocortex's response.

Considering that the amygdala's response occurs earlier than the neocortex's response by 100ms, the specific situations by icon application is classified into the following three cases in different conditions, which will be evaluated as follows.
<1> Icons used and not used
   This reaction can be grasped as a reaction by an interest to the icons. Thus, the amygdala's response appears first, and the sensory response precedes. It also shows not only interest but also recognition of necessity, etc.
<2> What is affected by the icons' area and the line component of the check sheet
   An examinee will apply ions while being influenced by the areas or the line components. That is, the presence or absence of an influence on the areas or the line components is an object of observation.

If the icon is applied not in an independent manner, not only the reaction by amygdala but also the reaction by neocortex is added later. In other words, while cerebral reactions require trial and error, selection and application is irreversible reaction, so as a result, it is observed that amygdala's response occurs first.

### <3> Interaction among icons

Icons can be observed not only in an independent manner but also in an interactive manner such as "overlapping" and "contact", and in some cases, icons may express the up-and-down or master-slave relationships.

The relationship between icons and line components of check sheets is the relationship between those that can be moved (icons) and those that cannot be moved (check sheets). In contrast, the icon-to-icon relationship differs only in that they can be moved, and because amygdala's response precedes neocortex's response by 100ms, this will be affected by the resulting icon selections, as well as the response described in the preceding section. In other words, the interaction among icons is that the meaning of the existence of an icon alone disappears and the relationship between the icons is created. For example, star icons and moon icons are perceived to be located at the upper part because they are in a celestial body. The combination of a round icon and a square icon recalls a doll from its shape.

The crowded arrangement means that the icons concentrate on a particular area or at a point. The crowded arrangement includes different stages of close-crowding, contact-crowding and overlap-crowding, which represents introversion, including a demand to hide oneself in a group. This reaction is derived from likes and dislikes, which will first be managed by amygdala.

The dispersive arrangement is a situation in which icons are not clustered and the distances between icons and line components remain constant. Dispersive Arrangement expresses openness and extroversion. Neocortex is reached as if there were no amygdala responses, such as "freely, without restraint...". In other words, Dispersive Arrangement is the response of neocortex.

The balanced arrangement is a case in which they are arranged in a well-balanced manner, such as one in each of the areas. This is a particular condition of the dispersive arrangement, which results from the neocortex's response. If neocortex has no sense of balance, the balanced arrangement cannot be formed.

Though not as much as the crowded arrangement, the deviative arrangement is a case in which the icons are concentrated slightly in a particular area. In other words, the deviative arrangement is a case in which the icon distribution is biased, which results preferentially from the amygdala's response. Neocortex has a little affection to the deviative arrangement, because it may be corrected later.

The bottom arrangement is a case in which the icons are located at the bottom of the virtual frame. The bottom arrangement is a stabilization-seeking reaction that is a response by neocortex response occurring later than amygdala.

The upper arrangement is a case in which the icons are concentrated in the upper three areas. The upper arrangement retains a natural sense, and its relation to the icons is important. For example, when the star and moon icons are located in the upper areas, it will represent a normal sense because they are in sky. If the star or moon icons are located in the lower areas, it can be assessed that there is some effect on neocortex. If many icons are placed on the upper areas, this represents anxiety. Anxiety is a neocortex's reaction, and it can be confirmed that this is much influenced by the later reaction.

The top-and-bottom arrangement is a case in which the icons are located in the upper and lower areas and there are few icons in the center. The top-and-bottom arrangement emerges as a result of being influenced by the likes and dislikes and a strange feeling. This will result in main from an amygdala's response.

The left-and-right arrangement is a case in which the icons are located in the left and right areas, which is a logical response by neocortex. The top-and-bottom arrangement and the left-and-right arrangement would be found in a limited number of cases.

The protruding arrangement is classified into complete out-of-virtual-frame protrusion and as partial protrusion. The complete protrusion is a reaction preferentially by amygdala, which may be understood that something in subconscious becomes explicit and then react. The partial protrusion is a reaction preferentially by neocortex rather than by amygdala, including hesitation.

The doll arrangement is a case in which the icon located in the lower area is square and the icon located in the upper area is circular. The doll arrangement is the reaction by neocortex, which something originally in subconscious becomes explicit.

Since every icon has its own means as shown in TABLE 3A and TABLE 3B, selecting an icon is interpreted as being introspective because it reflects own mental function and state.
On the other hand, it is understood as being reflective, if the icons are deemed to be arranged in some interactive manner with s specific position, angle and the like in the check sheet that is an environment. Similarly, the interaction among icons can be grasped as being reflective. In other words, "introspective" means a looking-back by oneself on the function and state of one's own mind, and "reflective" means that meanings and interpretations are given from outside without looking back by oneself on the function and state of one's own mind.

Because amygdala's responses occur earlier than neocortex's responses by 100ms, the sensory system reacts earlier than thinking, analyzing and judging by neocortex. In other words, retina reacts thorough visual information, which is transmit to amygdala. Then, also thanks to neocortex's response, the brain expresses a potential response. Icon application also involves bored response with time, but if interested or motivated, the response persists and the icon application increases. If there is a limit on the count-up number of the icons, neocortex will respond in a similar manner within the limits.

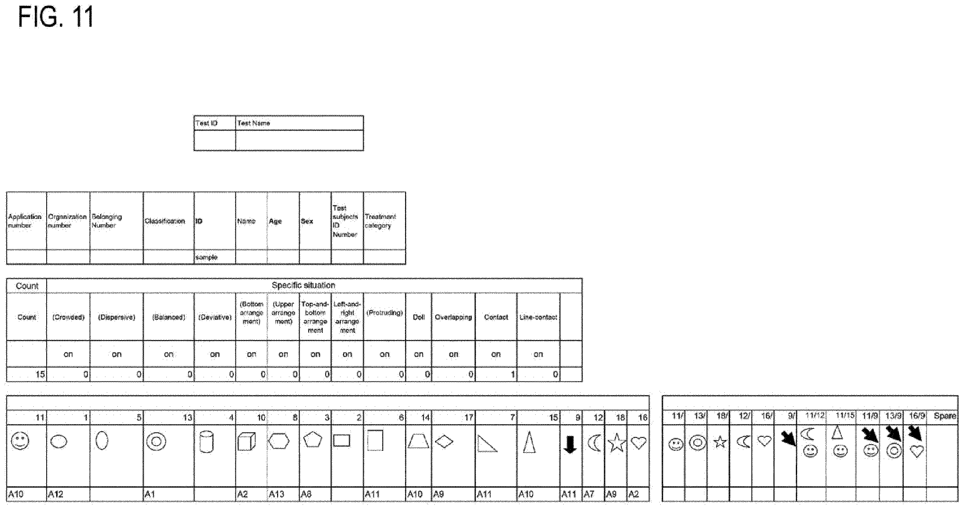

Fig. 11 shows the content of the process specification table stored in the storage means, and the processed result is output as a test result as follows.
Icon part (count: the icon number applied)
Icon part (specific situation)
Icon part (positional state of icon application)
Icon part (state of adopted icons alone and in combination)

The computer-processed data is stored in the headquarter computer as a test result according to the present invention.

The order of the icons shown in Fig. 11 will be described. In Fig. 11, the icons are shown in the order of from the rounded icons to the angular icons from left to right. Icon No. 12 (moon), Icon No. 18 (star), and Icon No. 16 (heart shape) are shown in this order from left to right.

### EXAMPLES

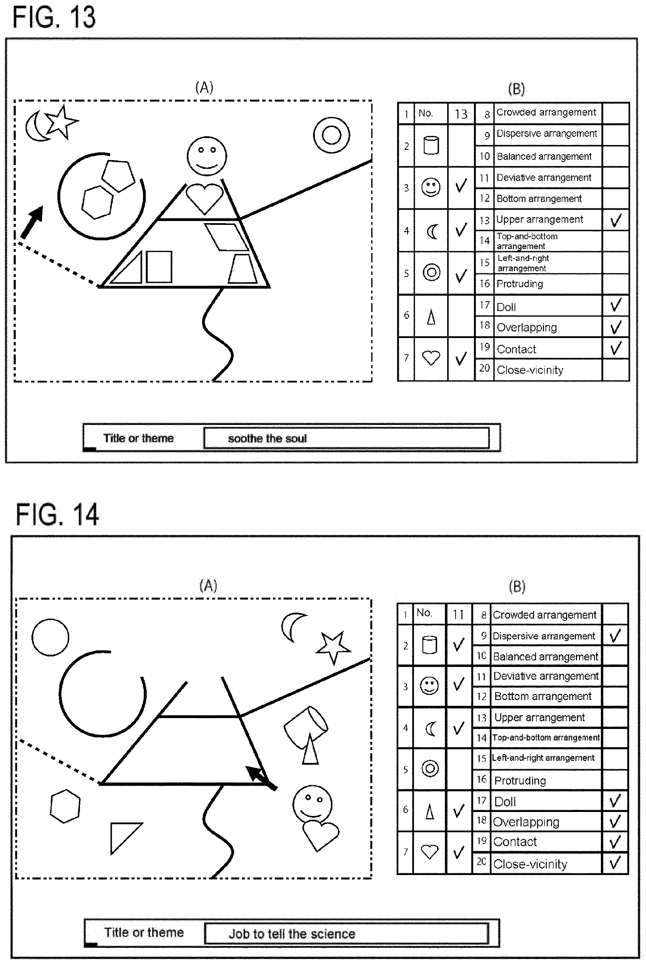

Figs. 13 to 17 show different examinees' examples. In each of the drawings, (A) shows an example in which an icon is applied by the examinee, (B) shows processing of the in-computer data obtained by totaling the locations of the applied icons.

In Fig. 13(A), the application state of the icons by the examinee 0001 will be recognized as follows based on TABLE 7.

**[TABLE 9]**

| | | | |
|---|---|---|---|
| A (count-up): 13 | | | |
| B (select specific icon) and its occurrences: | | | |
| Selected designation of specific icon: | | Location of specific situation appearance: | Definitions of Fig. 3A to Fig. 3L |
| Icon No. 11 (Face): | | Area A-5 : | Doll |
| Icon No. 12 (Moon): | | Area A-7 : | Released |
| Icon No. 13 (doughnut): | | Area A-9: | Released |
| Icon No. 16 (heart shape): | | Area A-4: | Manifestation of intentional objects |
| C-F (Arrangement designation, etc.): | | | |
| | Crowded arrangement :0 | | |
| | Dispersive arrangement :0 | | |
| | Balanced arrangement :0 | | |
| | Deviative arrangement :0 | | |
| | Bottom arrangement :0 | | |
| | Upper arrangement :1 | | |
| | Top-and-bottom arrangement :0 | | |
| | Left-and-right arrangement :0 | | |
| | Protruding arrangement: 0 | | |
| | Doll arrangement: 1 | | |
| | Overlapping :1 | | |
| | Contact :1 | | |
| | Line-contact :0 | | |

Therefore, at this stage, the examinee 0001 is determined as follows.

**[TABLE 10]**

| Selected designation of specific icon: | Appearance of specific situation | Definitions |
|---|---|---|
| (0001) | Location (Area) | |
| Count :13 | | Moderate motivation |
| Icon No. 11 (Face) | A5 | Not rejecting human relationships |
| Icon No. 13 (doughnut) | A9 | Strange feeling, weakness, etc. |
| Icon No. 12 (Moon) | A7 | Be able to make common sense decisions and thinkinging |
| Icon No. 16 (heart shape) | A4 | Be kind and be able to consider |
| Upper arrangement | | Be somewhat worried about the situation |
| Doll arrangement | | High capacity for storage, analysis, judgment, etc. |
| Overlapping | | Be able to maintain a degree of human relationships |
| Contact | | Be able to interact with others at reasonable distances |

In Fig. 14, part (A), the application state of the icons by the examinee 0002 will be recognized as follows based on TABLE 7.

**[TABLE 11]**

| | | | |
|---|---|---|---|
| A (count-up): 11 | | | |
| B (select specific icon) and its occurrences: | | | |
| Selected designation of specific icon: | | Location of specific situation appearance: | Definitions of Fig. 3A to Fig. 3L |
| Icon No. 4 (column): | | Area A-10 : | afford, no problem |
| Icon No. 11 (Face): | | Area A-11 : | Certain people are irrelevant |
| Icon No. 12 (Moon): | | Area A-9: | Released |
| Icon No. 15 (isosceles triangle): | | Area A-10: | Afford, no problem |
| Icon No. 16 (heart shape): | | Area A-11: | Feeling that no one will understand |
| Icon No. 9 (arrow): | | Contact point X-5 (intersection of line L9 and TAL17): | I realize that it is somehow bad for me. |
| C-F (Arrangement designation, etc.): | | | |
| | Crowded arrangement :0 | | |
| | Dispersive arrangement :1 | | |
| | Balanced arrangement :0 | | |
| | Deviative arrangement :0 | | |
| | Bottom arrangement :0 | | |
| | Upper arrangement :0 | | |
| | Top-and-bottom arrangement :0 | | |
| | Left-and-right arrangement :0 | | |
| | Protruding arrangement: 0 | | |
| | Doll arrangement: 1 | | |
| | Overlapping :1 | | |
| | Contact :1 | | |
| | Line-contact :1 | | |

Thus, the examinee 0002 is determined at this stage as follows.

**[TABLE 12]**

| Selected designation of specific icon: | Appearance of specific situation | Definitions |
|---|---|---|
| (0002) | Location (Area) | |
| Count :11 | | Somewhat lower desire |
| Icon No. 11 (Face) | A-10/11 | Not rejecting human relationships |
| Icon No. 12 (Moon) | A9 | Be able to make common sense decisions and thinkinging |
| Icon No. 16 (heart shape) | A11 | Be kind and be able to consider |
| Icon No. 4 (column) | A10 | It originally represents a sense of presence, but here is a reflecting telescope |
| Icon No. 15 (isosceles triangle) | A10 | Used as a balance stand (of a reflecting telescope) |
| Icon No. 4 (column) + | - | Icon No. 4 (column) and icon |
| Icon No. 15 (isosceles triangle) | - | Combinations with No.15 (isosceles triangle) represent something like reflective telescopes (not have deep meaning) |
| Dispersive arrangement | | Open, not persistent |
| Doll arrangement | | High capacity for storage, analysis, judgment, etc. |
| Overlapping | | Be able to maintain a degree of human relationships |
| Contact | | Be able to interact with others at reasonable distances |
| Line-contact | | I have a dependence. |

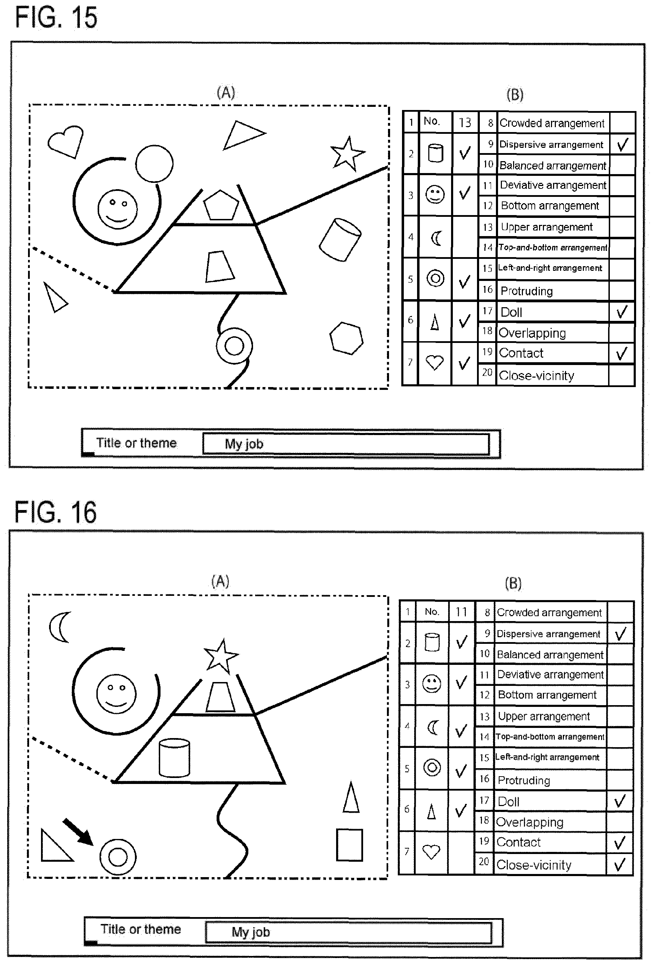

In Fig. 15, part (A), the application state of the icons by the examinee 0003 will be recognized as follows based on TABLE 7.

**[TABLE 13]**

| | | | |
|---|---|---|---|
| A (count-up): 13 | | | |
| B (select specific icon) and its occurrences: | | | |
| Selected designation of specific icon: | | Location of specific situation appearance: | Definitions of Fig. 3A to Fig. 3L |
| Icon No. 4 (column): | | Area A-10 : | Afford, no problem |
| | | Area A-1 : | Certain |
| Icon No. 11 (Face): | | | people are irrelevant. |
| Icon No. 13 (doughnut): | | Lines L-16: | |
| Icon No. 15 (isosceles triangle): | | Area A-13: | The sense of destruction and aggression do not vanish. |
| Icon No. 16 (heart shape): | | Area A-7: | Released |
| C-F (Arrangement designation, etc.): | | | |
| | Crowded arrangement :0 | | |
| | Dispersive arrangement :1 | | |
| | Balanced arrangement :0 | | |
| | Deviative arrangement :0 | | |
| | Bottom arrangement :0 | | |
| | Upper arrangement :0 | | |
| | Top-and-bottom arrangement :0 | | |
| | Left-and-right arrangement :0 | | |
| | Protruding arrangement: 0 | | |
| | Doll arrangement: 1 | | |
| | Overlapping :0 | | |
| | Contact :1 | | |

Thus, the examinee 0003 is determined at this stage as follows.

**[TABLE 14]**

| Selected designation of specific icon: | Appearance of specific situation | Definitions |
|---|---|---|
| (0003) | Location (Area) | |
| Count :13 | | Moderate motivation |
| Icon No. 11 (Face) | A-1 | Not rejecting human relationships |
| Icon No. 13 (doughnut) | A-11/12 | Strange feeling, weakness, etc. |
| Icon No. 4 (column) | A10 | It is interpreted as make somebody do |
| Icon No. 15 (isosceles triangle) | A13 | The inclination itself is interpreted as representing the test subject's instability . |
| Icon No. 16 (heart shape) | A7 | Be kind and be able to consider |
| Dispersive arrangement | | Open, not persistent |
| Doll arrangement | | High capacity for storage, analysis, judgment, etc. |
| Contact | | Interact with others at reasonable distances |

In Fig. 16, part (A), the application status of the icons by the examinee 0004 will be recognized as follows based on TABLE 7.

**[TABLE 15]**

| | | | |
|---|---|---|---|
| A (count-up): 11 | | | |
| B (select specific icon) and its occurrences: | | | |
| Selected designation of specific icon: | | Location of specific situation appearance: | Definitions of Fig. 3A to Fig. 3L |
| Icon No. 4 (column): | | Area A-3 : | I want to target something |
| Icon No. 11 (Face): | | Area A-1 : | I want to monopolize or target a particular person. |
| Icon No. 12 (Moon): | | Area A-7 : | Released |
| Icon No. 13 (doughnut): | | Area A-13: | Wheels, cars |
| Icon No. 15 (isosceles triangle): | | Area A-11: | The sense of destruction and aggression do not vanish. |
| Icon No. 9 (arrow): | | Area A-13 : | Be aware |
| C-F (Arrangement designation, etc.): | | | |
| | Crowded arrangement :0 | | |
| | Dispersive arrangement :1 | | |
| | Balanced arrangement :0 | | |
| | Deviative arrangement :0 | | |
| | Bottom arrangement :0 | | |
| | Upper arrangement :0 | | |
| | Top-and-bottom arrangement :0 | | |
| | Left-and-right arrangement :0 | | |
| | Protruding arrangement: 0 | | |
| | Doll arrangement: 1 | | |
| | Overlapping :0 | | |
| | Contact :1 | | |
| | Line-contact :1 | | |

Thus, the examinee 0004 is determined at this stage as follows.

**[TABLE 16]**

| Selected designation of specific icon: | Appearance of specific situation | Definitions |
|---|---|---|
| (0004) | Location (Area) | |
| Count :11 | | Distraction, excessive consciousness |
| Icon No. 11 (Face) | A-1 | Not rejecting human relationships |
| Icon No. 13 (doughnut) | A-12 | Strange feeling, weakness, etc. |
| Icon No. 4 (column) | A-3 | The presence without inclination in closed space represents a sense of loneliness |
| Icon No. 12 (Moon) | A-7 | Be able to make common sense decisions and thinking |
| Icon No. 15 (isosceles triangle) | A-10 | Have image of a sharp, dangerous, or sticking, etc. |
| Icon No. 6 (rectangle) | A-11 | It indicates unusual things. Differences from others, etc. |
| Icon No. 15 (isosceles triangle) | - | Associate a small house |
| + Icon No. 6 | | |
| Dispersive arrangement | | Open, not persistent |
| Doll arrangement | | High capacity for storage, analysis, judgment, etc. |
| Contact | | Interact with others at reasonable distances |
| Line-contact | | I have a dependence. |

In Fig. 17, part (A), the application state of the icons by the examinee 0005 will be recognized as follows based on TABLE 7.

**[TABLE 17]**

| | | | |
|---|---|---|---|
| A (count-up): 18 | | | |
| B (select specific icon) and its occurrences: | | | |
| Selected designation of specific icon: | | Location of specific situation appearance: | Definitions of Fig. 3A to Fig. 3L |
| Icon No. 4 (column): | | Area A-10 : | Afford, no problem |
| Icon No. 11 (Face): | | Area A-1 : | I want to monopolize or target a particular person. |
| Icon No. 12 (Moon): | | Area A-1 : | Coexistence with anxiety |
| Icon No. 13 (doughnut): | | Area A-1: | I want to target something |
| Icon No. 15 (isosceles triangle): | | Area A-4: | Dangerous elements that cannot be controlled |
| Icon No. 16 (heart shape): | | Area A-6: | Tight. |
| Icon No. 4 (column) | | Line A-10 | : Afford, no problem |
| C-F (Arrangement designation, etc.): | | | |
| | Crowded arrangement :0 | | |
| | Dispersive arrangement :0 | | |
| | Balanced arrangement :0 | | |
| | Deviative arrangement :0 | | |
| | Bottom arrangement :0 | | |
| | Upper arrangement :0 | | |
| | Top-and-bottom arrangement :0 | | |
| | Left-and-right arrangement :0 | | |
| | Protruding arrangement: 0 | | |
| | Doll arrangement: 0 | | |
| | Overlapping :0 | | |
| | Contact :1 | | |
| | Line-contact :0 | | |

Thus, the examinee 0005 is determined at this stage as follows.

**[TABLE 18]**

| Selected designation of specific icon: | Appearance of specific situation | Definitions |
|---|---|---|
| (0005) | Location (Area) | |
| Count :18 | | Distraction, excessive consciousness |
| Icon No. 11 (Face) | A-1 | Not rejecting human relationships |
| Icon No. 13 (doughnut) | A-1 | Strange feeling, weakness, etc. |
| Icon No. 4 (column) | A-10 | Be used as representing a strong dependence |
| Icon No. 15 (isosceles triangle) | A-4 | It represents a persistent dependence similarly |
| Icon No. 12 (Moon) | A-1 | Be able to make common sense decisions and thinking |
| Icon No. 16 (heart shape) | A-6 | Be kind and be able to consider |
| There is a large contact of icons. | | It indicates a strong dependence and a strong dependability |

Each of the above-described data shown in Figs. 13 to 17 is stored in the headquarter computer as the inspection result, which can be used as a material for further investigation.

The standard test time for icon application according to the present invention is about 5 minutes. In addition, there are restrictions on the age of examinees, and those who is between the workable age and 60 years old are eligible for inspection, especially those who are up to about 45 years old.

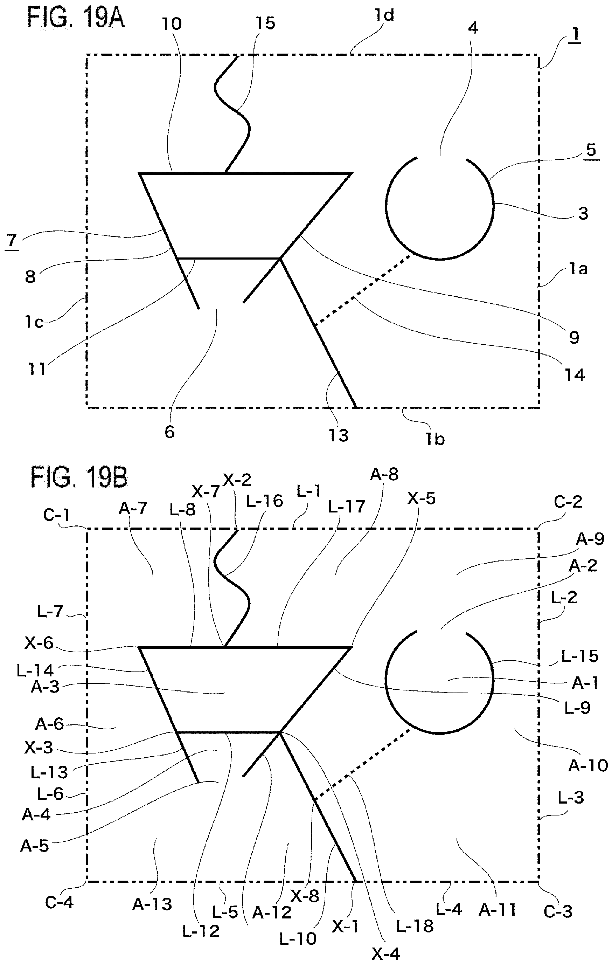

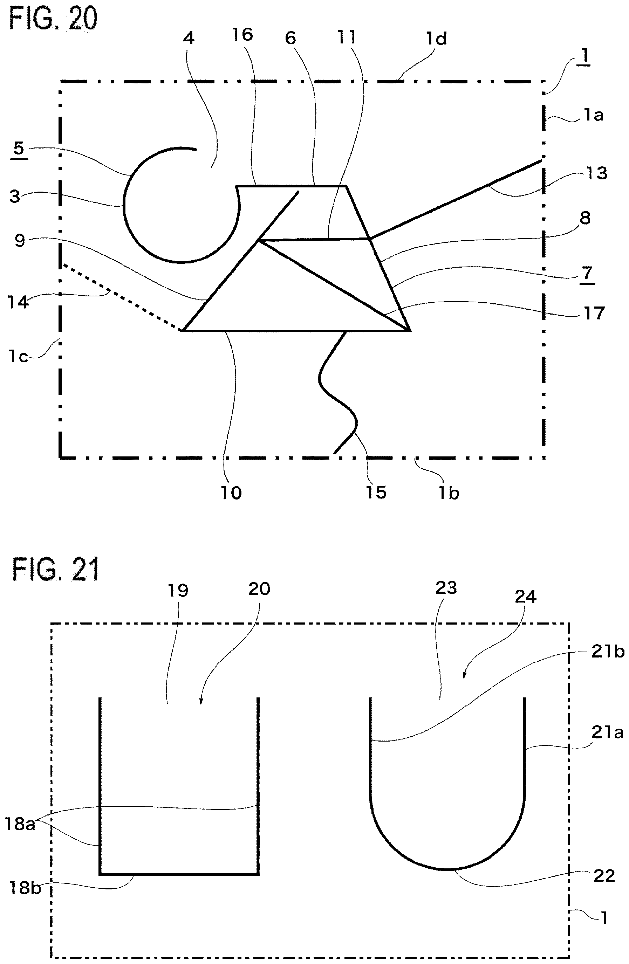

The present invention is not limited to the above-described embodiments. For example, the shape of the figure in which the areas are partitioned is not limited to that shown in Fig. 1 and may include various embodiments such as shown in Fig. 19A and Fig. 20, for example.

In Fig. 19A, the boundary elements depicted in a rectangular window 1 consisting of window virtual lines 1a to 1d comprises a circular figure 5 having a cut-off 4 at the upper part of a curved circumferential line 3, a triangular figure 7 having a cut-off 6 at the apex thereof and an intermediate straight line 11 extending in parallel with a straight bottom line 10 between opposite oblique-side lines 8, 9, a straight line 13 (inclined straight line) extending obliquely down and to right from the right end of the intermediate line 11 to the window virtual line 1b, a broken line 14 (inclined broken line) extending obliquely up and to right from the middle portion of the inclined straight line 13 to the lower part of the circular figure 5, and a S-shape curved line 15 extending up from the middle portion of the bottom 10 to the window virtual line 1d. With these boundary elements, the above-mentioned areas are divided into nine sectional areas, among which three are located almost in a horizontal direction and other three almost in a vertical direction. The cut-off 4 pens upward and the cut-off 6 opens downward.

In Fig. 20, the boundary elements depicted in a rectangular window 1 consisting of window virtual lines 1a to 1d comprises a circular figure 5 having a cut-off 4 at the upper-right part of a curved circumferential line 3, a triangular figure 7 having a cut-off 6 at the apex thereof and an intermediate straight line 11 extending in parallel with a straight bottom line 10 between opposite oblique-side lines 8, 9, a straight line 13 (inclined straight line) extending obliquely up and to right from the right end of the intermediate line 11 to the window virtual line 1a, a broken line 14 (inclined broken line) extending obliquely up and to left from the left end (opposite to the inclined straight line 13) of the bottom 10 to the window virtual line 1c, a S-shape curved line 15 extending down from the rightward middle portion of the bottom 10 to the window virtual line 1b, another straight line 16 (connecting line) connecting one end of the cut-out 4 of the circular figure 5 to one end of the cut-out 6 of the triangular figure 7, and another straight line 17 (inclined line) extending obliquely between the left end (opposite to the inclined straight line 13) of the intermediate line 11 and the right end of the bottom 10. With these boundary elements, the areas to be described in detail below are divided into nine sectional areas, among which three are located almost in a horizontal direction and other three almost in a vertical direction. Both cut-offs 4 and 6 opens upward.

Fig. 21 shows another embodiment wherein only a part of the above-mentioned boundary elements is used. In this embodiment, the boundary elements depicted in the window 1 comprise a quadrangular hole 20 at the left having straight lines 18a and a straight line 18b perpendicular thereto with an opening at the top 19, and a U-shaped hole 24 at the right having parallel straight lines 21a, 21b and a semi-circular curved line 22 connecting the lower ends of the straight lines 21a, 21b with an opening at the top 23.

In the case of the check sheet as shown in Fig. 21, a relatively simple investigation is performed, which makes it possible to reduce the burden on the examinee. On the other hand, when a check sheet in which a lot of the boundary elements are depicted to form a lot of areas as shown in Figs. 1, 19A and 20, the investigation becomes relatively complicated, which makes it possible to perform a wide range of investigations and therefore to obtain various analytical results.

### Industrial Applicability

The visual approach-based aptitude testing system according to the present invention can be used, for example, for judgement of vocational aptitude of personnel in entrance examinations (including mid-career recruitment examinations) and promotion examinations in companies and government offices. It may also be used for personnel evaluation.

### Reference Signs List

1 window
1a window virtual line
1b window virtual line
1c window virtual line
1d window virtual line
3 curved line
4 cut-off
5 circular figure
6 cut-off
7 triangular figure
8 oblique-side line
9 oblique-side line
10 bottom
11 intermediate line
13 inclined straight line
14 inclined broken line
15 curved line
16 connecting line
17 inclined straight line
18a straight line
18b straight line
19 top
20 hole
21 straight line
22 curved line
23 top
24 U-shaped hole

## Claims

1. A visual approach-based aptitude testing system comprising a headquarter computer connected to an examinee's terminal through a communication network;
said headquarter computer includes:
means for providing, to said examinee's terminal, a check sheet in which a plurality of areas are formed by boundary elements depicted in a frameless test area and which comprises graphic figures having no particular meaning by itself, and a plurality of icons applicable to said check sheet;
storage means for storing table 1 which predetermines the definition of said boundary elements in said check sheet, table 2 which predetermines the kind, number and definition of said icons, and table 3 which predefines the meaning of position of said icon in said check sheet;
writing means for sequentially writing, to said storage means, test data including said icons applied by said examinee and an examinee's ID transmitted from said examinee's terminal;
count-up execution means for sequentially reading out said test data and said examinee's ID stored in said storage means to count the total number of said icons;
means for executing discrimination whether or not the arrangement of said icons in said read-out test data falls under said definition in each tables,
wherein the results of said discrimination are stored in said headquarter computer and used for evaluation of the examinee's aptitude based on the total number of said icons in a test object and the overall shape of the test object.

2. A visual approach-based aptitude testing system according to claim 1 wherein said boundary elements comprise lines and/or corners.

3. A visual approach-based aptitude testing system according to claim 2 wherein said lines comprise straight lines, curved lines and broken lines or any combinations thereof.

4. A visual approach-based aptitude testing system according to claim 2 or 3 wherein said corners comprise corners of virtual frame, and/or points of intersection between lines.

5. A visual approach-based aptitude testing system according to any one of claims 1 to 4 wherein said storage means further stores table 4 which predefines the meaning of positional relationship (hereinlater referred to by "icon relationship") between a plurality of said icons or between said icon and said line, and said headquarter computer further comprises means for executing discrimination whether or not said icon relationship exists in the arrangement of said icons in said read-out test data, in reference to said definition in each of said tables.

6. A visual approach-based aptitude testing system according to any one of claims 1 to 5 wherein said storage means further stores table 5 which predefines the meanings of said applied icons forming a specific pattern, and said headquarter computer further comprises means for executing discrimination whether or not said predefined specific pattern exists.

7. A visual approach-based aptitude testing system according to any one of claims 1 to 6 wherein said storage means further stores table 6 which predefines the meaning of appearance of a specific one of said icons, and said headquarter computer further comprises means for executing discrimination whether or not said specific icon appears.

8. A visual approach-based aptitude testing system according claim 6 wherein said specific pattern comprises crowded arrangement, dispersive arrangement, balanced arrangement, deviative arrangement, bottom arrangement, upper arrangement, top-and-bottom arrangement, left-and-right arrangement, protruding arrangement and /or doll arrangement.

9. A visual approach-based aptitude testing system according claim 5 wherein said icon relationship comprises overlapping, contact, line-contact and/or close-vicinity.
